# EUROPEAN PATENT APPLICATION

(11) **EP 3 345 901 A2**
(43) Date of publication of application: **11.07.2018**
(21) Application number: 17202691.6
(22) Date of filing: 13.04.2012
(51) Int. Cl.: C07D 403/06, C07D 403/12, C07D 417/04, C07D 311/28

(54) **SMALL MOLECULES AS ANTIAGING AGENTS**

(30) Priority: 29.06.2011 US 201161502588 P
(62) Divisional of application: 12804280.1
(71) Applicant: President and Fellows of Harvard College, Cambridge, MA 02138 (US); Mayo Foundation for Medical Education and Research, Rochester, Minnesota 55905 (US)
(72) Inventor: Sinclair, David A., Chestnut Hill, MA 02467 (US); Price, Nathan L., Cambridge, MA 02139 (US); Chini, Eduardo, Rochester, MN 55902 (US); Clardy, Jon C., Jamaica Plain, MA 02130 (US); Cao, Shugeng, Waltham, MA 02452 (US)
(74) Representative: Helbig, Christian

(57) **Abstract**

The application provides an agent selected from nicotinamide riboside, tryptophan, quinolinic acid, nicotinamide, nicotinamide mononucleotide, and nicotinic acid for use in:
i) retarding, treating, or preventing an age-related disease in a subject; or
ii) retarding aging in a subject; or
iii) imparting the benefits of diet or exercise to a subject.

## Description

### RELATED APPLICATIONS

This application claims the benefit of priority to United States Provisional Patent Application serial number 61/502,588, filed June 29, 2011, the contents of which are hereby incorporated by reference.

### GOVERNMENT SUPPORT

This invention was made with Government support under National Institutes of Health award AG028730. The Government has certain rights in the invention

### BACKGROUND

Nicotinamide adenine dinucleotide (NAD⁺) was originally discovered as a cofactor in redox reactions but has recently emerged as a small molecule regulator of many other processes including signaling pathways, cell-cell communication, and epigenetic changes. Once thought to be very stable, levels of NAD⁺ rise in response to dieting and exercise, and are a key component of the 24-hour circadian rhythm. Conversely, obesity and aging reduce NAD⁺ levels. A major player in this system is Nampt, an enzyme that responds to diet and circadian clock, and protects cells from apoptosis.

Downstream mediators of NAD⁺ signaling are the sirtuins, which are NAD⁺-dependent deacetylases and mono-ADP ribosyltransferases. The sirtuins were originally discovered in yeast as longevity genes, but are now known to control many aspects of mammalian physiology, including the health benefits of reduced calorie intake and exercise. Sirtuins target many well-known physiological regulators such as PGC-1alpha, p53, and NF-kB.

There are seven mammalian sirtuins, SIRT1-7. Increasing the expression or the activity of SIRT1 -- by genetic means or with small molecule SIRT1-activating compounds (STACs) -- imparts broad health benefits in mammals by mimicking dieting and exercise, including the prevention of obesity, liver steatosis, cardiovascular diseases, and insulin resistance/type II diabetes. In addition, SIRT1 and its activating molecules have been shown to prevent or treat numerous other diseases of aging including many types of cancer, cataracts, bone loss, stroke, inflammatory disorders (e.g. of the gut and lungs; COPD), neurodegeneration, and even memory consolidation.

Less is known about the other sirtuins, but activating SIRT2, SIRT3 and SIRT6 (and possibly other sirtuins) seem to be useful avenues for treating diseases. SIRT2 controls the cell cycle and senescence, therefore activating it may help with diseases that result from cell cycle defects and senescence, such as skin aging, and cancer. SIRT3 controls mitochondrial activity, by deacetylation of a number of electron transport chain components and antioxidant enzymes.

In addition to the sirtuins, poly-ADP-ribosyltransferases (PARPs) control DNA repair and cell survival. The PARP reaction, adding ADP riboses to proteins, ensures proper repair of damaged DNA. However, high PARP activity can deplete the cell of NAD⁺, resulting in reduced sirtuin activity, cell dysfunction, and cell death.

CD38 is a type II transmembrane glycoprotein that was initially identified as a surface antigen in lymphocytes. It is ubiquitously expressed in mammalian tissues both on the cell surface and in the nuclear membrane. CD38 possesses ligand-stimulated signaling functions in addition to multiple enzymatic activities, including the ability to synthesize the second messengers cADPR, ADPR, and NAADP, involved in calcium mobilization.

Recent studies, however, have shown that the primary enzymatic activity of CD38 is the hydrolysis of NAD⁺, resulting in the production of NAM and ADPR. This enzymatic function appears to be independent of its ligand-stimulated activity. Importantly, mice lacking CD38 show almost no NADase activity in most tissues and have tissue NAD⁺ levels 10 to 20 fold higher than wildtype animals.

Cells from CD38 KO mice show increased NAD+ levels. Additionally, the mice are protected from weight gain and loss of glucose homeostasis in a model of diet induced obesity. Small molecule inhibitors of CD38 could impart the health benefits of caloric restriction and exercise, among others.

### SUMMARY OF THE INVENTION

In certain embodiments, the invention relates to a compound of formula **I** or a compound of formula **II**: 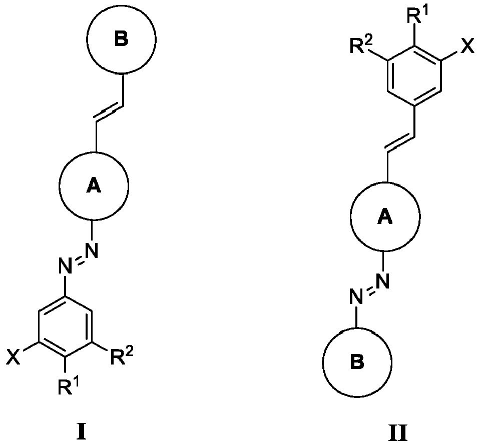 wherein 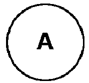
is an aryl heterocycle diradical; 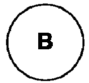
is heteroaryl;
X is halo;
R¹ is hydroxy, alkoxy, or amino; and
R² is hydroxy, alkoxy, or amino.

In certain embodiments, the invention relates to a compound of formula **III**: 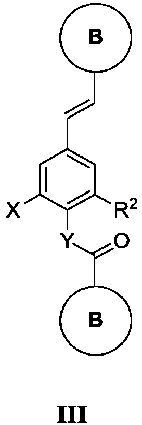 wherein, independently for each occurrence, 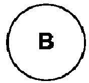
is heteroaryl;
X is halo;
R² is hydroxy, alkoxy, or amino; and
Y is -O- or -NH-.

In certain embodiments, the invention relates to a compound of formula **IV**: 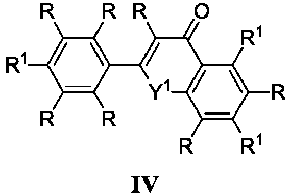 wherein, independently for each occurrence,
R is -H, halo, aryl, nitro, alkyl, hydroxy, alkoxy, or amino;
R¹ is hydroxy, alkoxy, or amino; and
Y¹ is -S-, -O-, or -NH-.

In certain embodiments, the invention relates to a compound of formula **V**: 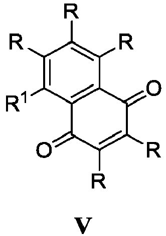 wherein, independently for each occurrence,
R is -H, halo, aryl, nitro, alkyl, hydroxy, alkoxy, or amino; and
R¹ is hydroxy, alkoxy, or amino.

In certain embodiments, the invention relates to a compound of formula **VI**: 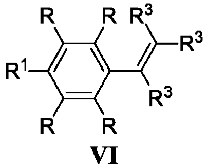 wherein, independently for each occurrence,
R is -H, halo, aryl, nitro, alkyl, hydroxy, alkoxy, or amino;
R¹ is hydroxy, alkoxy, or amino;
R³ is -H, cyano, -CO₂R⁴, or -C(O)N(R⁴)₂; and
R⁴ is -H or alkyl.

In certain embodiments, the invention relates to a compound of formula **VII**: 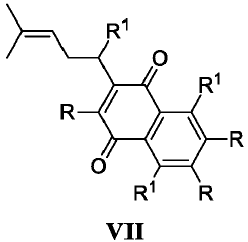 wherein, independently for each occurrence,
R is -H, halo, aryl, nitro, alkyl, hydroxy, alkoxy, or amino; and
R¹ is hydroxy, alkoxy, or amino.

In certain embodiments, the invention relates to a compound of formula **VIII**: 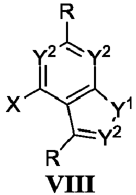 wherein, independently for each occurrence,
R is -H, halo, aryl, nitro, alkyl, hydroxy, alkoxy, or amino;
X is halo;
Y¹ is -O-, -S-, or -NH-; and
Y² is =N- or =CR-.

In certain embodiments, the invention relates to a compound of formula **IX**: 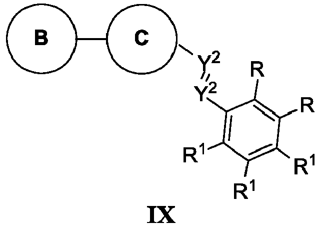 wherein, independently for each occurrence, 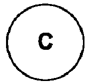
is a five-membered, unsaturated heterocycle diradical; 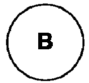
is heteroaryl;
R is -H, halo, aryl, nitro, alkyl, hydroxy, alkoxy, or amino;
R¹ is hydroxy, alkoxy, or amino; and
Y² is =N- or =CR-.

In certain embodiments, the invention relates to a compound of formula **X**: 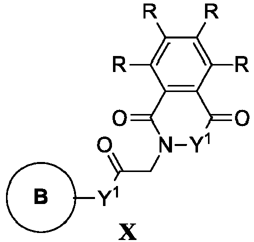 wherein, independently for each occurrence, 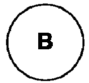
is heteroaryl;
R is -H, halo, aryl, nitro, alkyl, hydroxy, alkoxy, or amino;
Y¹ is -S-, -O-, or -NH-.

In certain embodiments, the invention relates to a compound of formula **XI**: 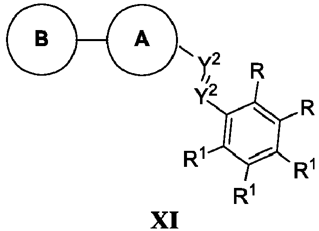 wherein, independently for each occurrence, 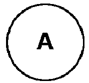
is an aryl heterocycle diradical; 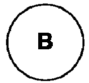
is heteroaryl;
R is -H, halo, aryl, nitro, alkyl, hydroxy, alkoxy, or amino;
R¹ is hydroxy, alkoxy, or amino; and
Y² is =N- or =CR-.

In certain embodiments, the invention relates to a compound of formula **XII**: 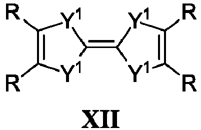 wherein, independently for each occurrence,
R is -H, halo, aryl, nitro, alkyl, hydroxy, alkoxy, or amino; and
Y¹ is -S-, -O-, or -NH-.

In certain embodiments, the invention relates to a compound of formula **XIII**: 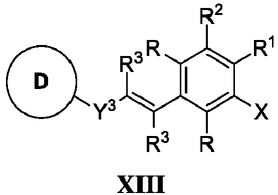 wherein, independently for each occurrence, 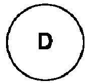
is aryl or heteroaryl;
R is -H, halo, aryl, nitro, alkyl, hydroxy, alkoxy, or amino;
R¹ is hydroxy, alkoxy, or amino;
R² is hydroxy, alkoxy, or amino;
R³ is -H, cyano, -CO₂R⁴, or -C(O)N(R⁴)₂;
X is halo;
Y³ is a bond, -C(O)-d, -C(O)NH-d, -NH-C(O)-d, or -C(O)NH-CH₂-d; and
d is a bond to

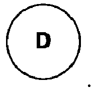

In certain embodiments, the invention relates to a compound of formula **XIV**: 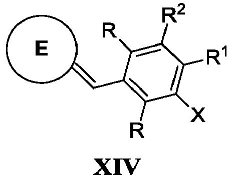 wherein, independently for each occurrence, 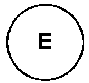
is a five-membered heterocycle radical;
R is -H, halo, aryl, nitro, alkyl, hydroxy, alkoxy, or amino;
R¹ is hydroxy, alkoxy, or amino;
R² is hydroxy, alkoxy, or amino; and
X is halo.

In certain embodiments, the invention relates to a compound of formula **XV**: 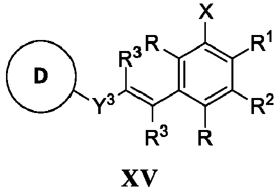 wherein, independently for each occurrence, 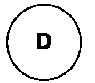
is aryl or heteroaryl;
R is -H, halo, aryl, nitro, alkyl, hydroxy, alkoxy, or amino;
R¹ is hydroxy, alkoxy, or amino;
R² is hydroxy, alkoxy, or amino;
R³ is -H, cyano, -CO₂R⁴, or -C(O)N(R⁴)₂;
X is halo;
Y³ is a bond, -C(O)-d, -C(O)NH-d, -NH-C(O)-d, or -C(O)NH-CH₂-d; and
d is a bond to

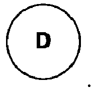

In certain embodiments, the invention relates to a method of inhibiting CD38 in a cell, comprising
contacting the cell with a therapeutically effective amount of an agent.

In certain embodiments, the invention relates to a method of inhibiting NADase activity in a cell, comprising
contacting the cell with a therapeutically effective amount of an agent.

In certain embodiments, the invention relates to a method of increasing NAD levels in a cell, comprising
contacting the cell with a therapeutically effective amount of an agent.

In certain embodiments, the invention relates to a method of increasing NAD⁺ levels in a cell, comprising
contacting the cell with a therapeutically effective amount of an agent.

In certain embodiments, the invention relates to a method of activating a PARP in a cell, comprising
contacting the cell with a therapeutically effective amount of an agent.

In certain embodiments, the invention relates to a method of treating a subject, comprising
administering to a subject in need thereof a therapeutically effective amount of an agent.

In certain embodiments, the invention relates to a method of retarding, treating, or preventing an age-related disease in a subject, comprising
administering to a subject in need thereof a therapeutically effective amount of an agent.

In certain embodiments, the invention relates to a method of retarding aging in a subject, comprising
administering to a subject in need thereof a therapeutically effective amount of an agent.

In certain embodiments, the invention relates to a method of imparting the benefits of diet and exercise to a subject, comprising
administering to a subject in need thereof a therapeutically effective amount of an agent.

### BRIEF DESCRIPTION OF THE FIGURES

**Figure 1** depicts (A.) Dose dependent inhibition of CD38 activity by exemplary compounds identified as CD38 inhibitors from known bioactive and commercial libraries. (B.) Structures of exemplary CD38 inhibitors.
**Figure 2** depicts (A.) Inhibition in cellular NADase activity by CD38 inhibitors Apigenin and Quercetin. (B and C.) NAD levels in WT (B.) and CD38 KO (C.) MEFs before and after 6 h treatment with CD38 inhibitor Apigenin.
**Figure 3** depicts (A.) Dose dependent inhibition of CD38 activity by and structures of (B.) commercial compounds structurally similar to proposed structure of novel inhibitor. (C. and D) Proposed structures (C.) and synthesis scheme (D.) for novel CD38 inhibitors.

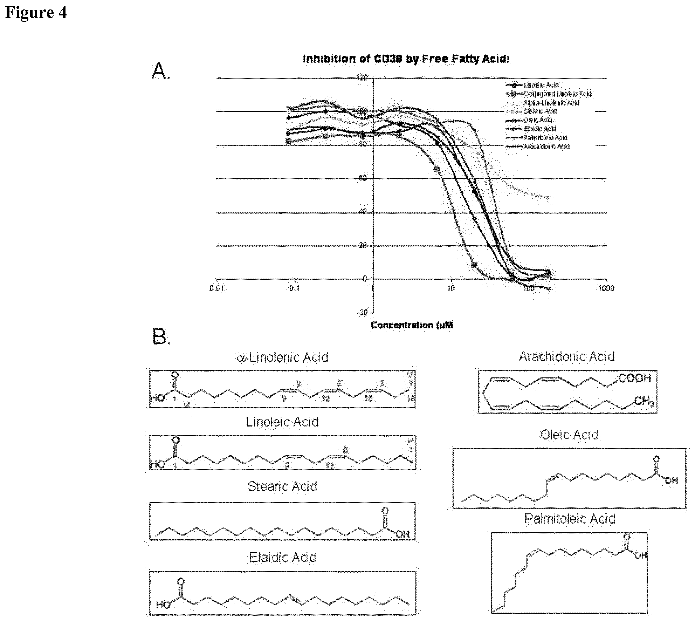

**Figure 4** depicts (A) dose dependent inhibition of CD38 activity by unsaturated free fatty acids. (B) Structures of free fatty acid CD38 inhibitors.
**Figure 5** depicts pathways for the synthesis and degradation of NAD⁺ in mammalian cells.
**Figure 6** depicts examples of soluble precursors to NAD⁺ as agents to raise cellular NAD⁺ levels and boost cellular energetics in damaged and/or aged cells.

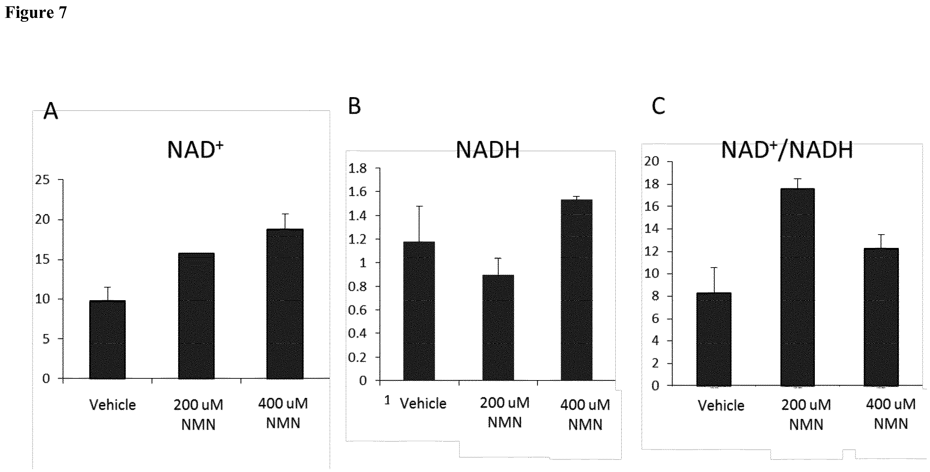

**Figure 7** depicts the increase in cellular NAD⁺ and NAD+/NADH in murine oogonial stem cells (OSCs) by nicotinamide mononucleotide. Oogonial stem cells were isolated from dissociated ovaries using a FACS based sorting protocol to purify OSCs free of contaminating oocytes (White et al., in press). Cells were maintained in culture medium consisting of minimum essential medium α (MEMα), 10% FBS, 1 mM sodium pyruvate, 1 mM non-essential amino acids, 2 mM 1-glutamine, 0.1 mM β-mercaptoethanol (Sigma), 10 ng/mL-1 LIF (Millipore), 1X N-2 MAX Media Supplement (R&D) 10 ng/mL EGF (Epidermal growth factor, Recombinant human; Gibco), 40 ng/mL human GDNF (glial cell line-derived neurotrophic factor; R&D systems), 1 ng/mL human bFGF (basic fibroblast growth factor; Gibco).
**Figure 8** depicts the increase in mitochondrial DNA content in murine OSCs by nicotinamide mononucleotide. Total cellular DNA was isolated from cells at the indicated time points using DNeasy Blood & Tissue Kit (Qiagen) according to the manufacturer's instructions. Mt DNA copy number was quantified using LightCycler 480 SYBR Green I Master (Roche Applied Science) using a Roche 480 PCR machine.
**Figure 9** depicts the increase in spontaneuous oocyte formation in cultured murine oogonial stem cells by nicotinamide mononucleotide. For assessment of spontaneous oocyte formation, each well of a 24- well plate was seeded with 25.000 OSCs, and the number of oocytes formed and released into the medium per well was assessed the second day after seeding as well as the designated time points after NMN treatment. Left bar = vehicle; middle bar = 200 µM nicotinamide mononucleotide (NMN); right bar = 400 µM NMN.
**Figure 10** depicts that the NAD+ precursor NMN raises NAD⁺ levels in vivo in young and old mice. Cardiac [NAD⁺] declines with age and is reversed by NMN treatment (n = 3; 200 mg/kg/d intraperitoneally for 1 week).
**Figure 11** depicts the restorative effects of an NAD⁺ precursor (NMN) on mitochondrial function in vivo. The decline in mitochondrial function in skeletal muscle of 24-month old mice is completely reversed by NMN (nicotinamide mononucleotide) after only 1 week of treatment (**A, B**). NMN is delivered by intraperitoneal (I. P.) injection and raises NAD⁺ levels in brain, heart and skeletal muscle ∼30-100%. NMN increases mitochondrial function in C2C12 cells in a SIRT1-dependent manner (**C**, **D**). sh Ctl = scrambled shRNA, Sh SIRT1 = shRNA against SIRT1.
**Figure 12** depicts weight gain in mice fed OpenStandard Diet with various compounds of the invention or a known activator of SIRT1 as a positive control (SRT1720, *N*-[2-[3-(piperazin-1-ylmethyl)imidazo[2,1-b][1,3]thiazol-6-yl]phenyl]quinoxaline-2-carboxamide). N = 12 mice per group. Diamonds = control (OpenStandard Diet only); squares = OpenStandard Diet containing SRT1720 (2 g/kg of food); triangles = OpenStandard Diet containing luteolin (0.5 g/kg of food); x = OpenStandard Diet containing apigenin (0.5 g/kg of food).
**Figure 13** depicts daily food consumption of mice fed OpenStandard Diet with various compounds of the invention or a known activator of SIRT1 as a positive control (SRT1720). N = 12 mice per group. SRT1720 = OpenStandard Diet + 2 g SRT1720/kg of food; Luteolin = OpenStandard Diet + 0.5 g luteolin/kg of food; Apigenin = OpenStandard Diet + 0.5 g apigenin/kg of food.
**Figure 14** depicts total NAD (top) and NADH (bottom) levels in ovaries. For each experiment, 3 ovaries were used.

### DETAILED DESCRIPTION OF THE INVENTION

### Overview

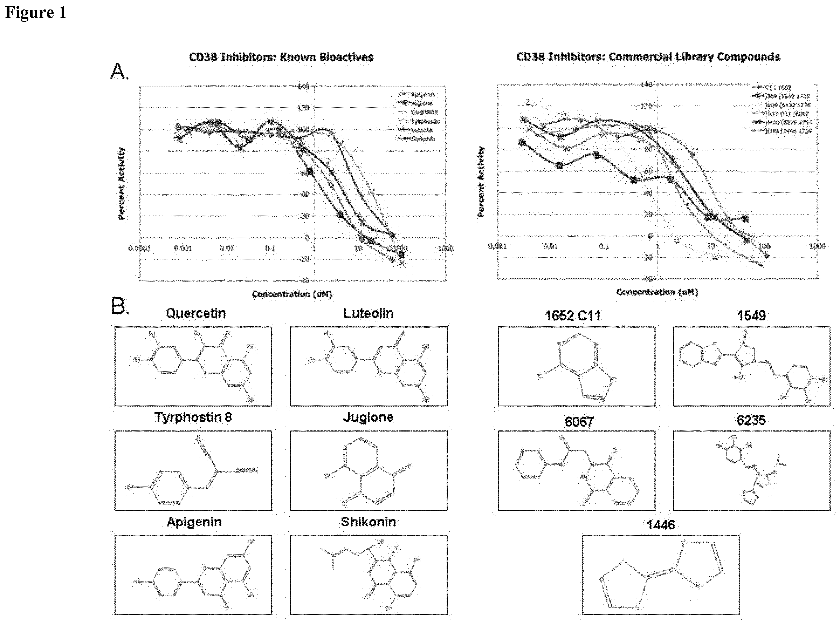

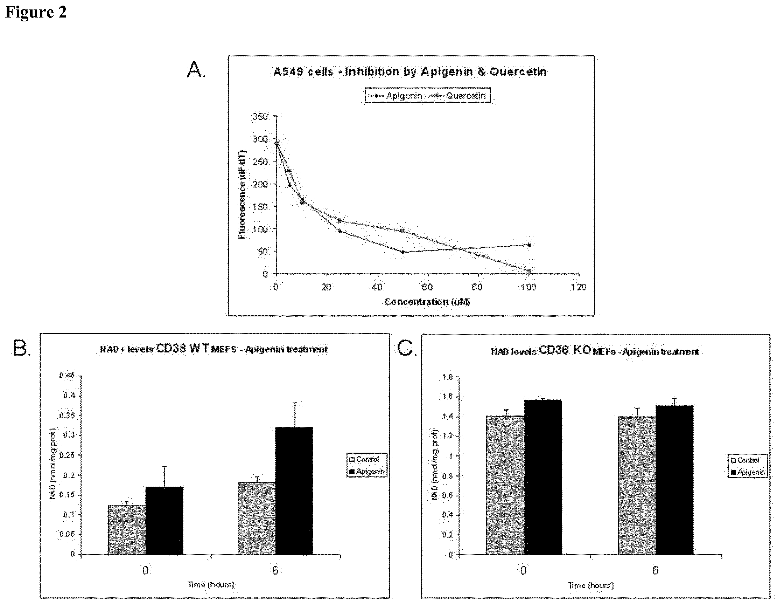

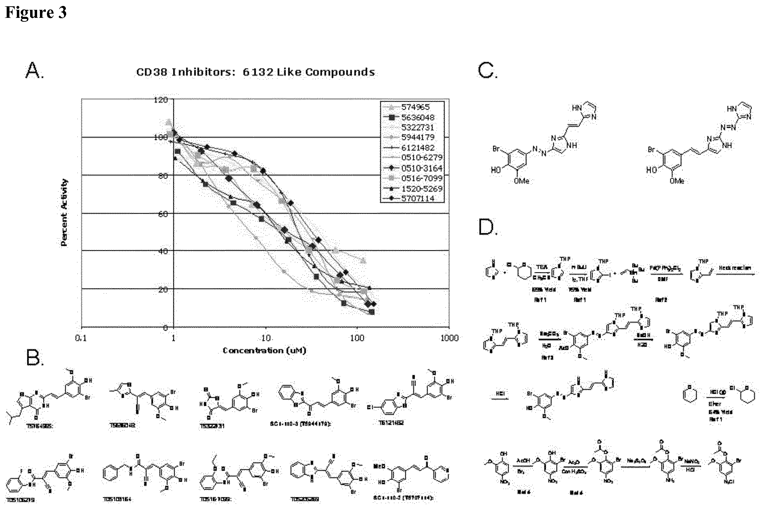

In certain embodiments, the invention relates to small molecules capable of inhibiting CD38 activity at nanomolar or micromolar concentrations (Figure 1 and Figure 3). Preliminary cellular assays on some of these compounds have shown that they are able to inhibit cellular NADase activity resulting in increased cellular NAD levels (Figure 2a and Figure 2b). Importantly CD38 knockout MEFs do not show any additional increase in NAD levels following treatment with CD38 inhibitors (Figure 2c).

In certain embodiments, the small molecule CD38 inhibitors (SMOCDIs) described herein, or derivatives thereof, provide a means of activating NAD+-dependent processes, thus imparting the benefits of dieting and exercise, as well as retarding age-related diseases, such as neurodegeneration, metabolic diseases, osteoporosis, inflammatory disorders (COPD, arthritis, psoriasis), cataracts, bone loss, and diseases resulting from mitochondrial dysfunction.

### Definitions

As used herein, the following terms and phrases shall have the meanings set forth below. Unless defined otherwise, all technical and scientific terms used herein have the same meaning as commonly understood to one of ordinary skill in the art to which this invention belongs.

The singular forms "a," "an," and "the" include plural reference unless the context clearly dictates otherwise.

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. The activity of such agents may render it suitable as a "therapeutic agent" which is a biologically, physiologically, or pharmacologically active substance (or substances) that acts locally or systemically in a subject.

"Diabetes" refers to high blood sugar or ketoacidosis, as well as chronic, general metabolic abnormalities arising from a prolonged high blood sugar status or a decrease in glucose tolerance. "Diabetes" encompasses both the type I and type II (Non Insulin Dependent Diabetes Mellitus or NIDDM) forms of the disease. The risk factors for diabetes include the following factors: waistline of more than 40 inches for men or 35 inches for women, blood pressure of 130/85 mmHg or higher, triglycerides above 150 mg/dl, fasting blood glucose greater than 100 mg/dl or high-density lipoprotein of less than 40 mg/dl in men or 50 mg/dl in women.

The term "ED₅₀" is art-recognized. In certain embodiments, ED₅₀ means the dose of a drug which produces 50% of its maximum response or effect, or alternatively, the dose which produces a pre-determined response in 50% of test subjects or preparations. The term "LD₅₀" is art-recognized. In certain embodiments, LD₅₀ means the dose of a drug which is lethal in 50% of test subjects. The term "therapeutic index" is an art-recognized term which refers to the therapeutic index of a drug, defined as LD₅₀/ED₅₀.

The term "insulin resistance" refers to a state in which a normal amount of insulin produces a subnormal biologic response relative to the biological response in a subject that does not have insulin resistance.

An "insulin resistance disorder," as discussed herein, refers to any disease or condition that is caused by or contributed to by insulin resistance. Examples include: diabetes, gestational diabetes, obesity, metabolic syndrome, insulin-resistance syndromes, syndrome X, insulin resistance, high blood pressure, hypertension, high blood cholesterol, dyslipidemia, hyperlipidemia, dyslipidemia, atherosclerotic disease including stroke, coronary artery disease or myocardial infarction, hyperglycemia, hyperinsulinemia and/or hyperproinsulinemia, impaired glucose tolerance, delayed insulin release, diabetic complications, including coronary heart disease, angina pectoris, congestive heart failure, stroke, cognitive functions in dementia, retinopathy, peripheral neuropathy, nephropathy, glomerulonephritis, glomerulosclerosis, nephrotic syndrome, hypertensive nephrosclerosis some types of cancer (such as endometrial, breast, prostate, and colon), complications of pregnancy, lipodystrophy, cholesterol related disorders, such as gallstones, cholescystitis and cholelithiasis, gout, obstructive sleep apnea and respiratory problems, osteoarthritis, and prevention and treatment of bone loss, e.g. osteoporosis.

The term "isolated" as used herein with respect to nucleic acids, such as DNA or RNA, refers to molecules separated from other DNAs, or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

The phrase "nucleic acid corresponding to a gene" refers to a nucleic acid that can be used for detecting the gene, e.g., a nucleic acid which is capable of hybridizing specifically to the gene.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. *See* Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases. Databases with individual sequences are described in Methods in Enzymology, ed. Doolittle, supra*.* Databases include Genbank, EMBL, and DNA Database of Japan (DDBJ).

"Obese" individuals or individuals suffering from obesity are generally individuals having a body mass index (BMI) of at least 25 or greater. Obesity may or may not be associated with insulin resistance.

"Replicative life span" which is used interchangeably herein with "life span" or "lifespan" of a cell refers to the number of daughter cells produced by an individual "mother cell." "Chronological aging," on the other hand, refers to the length of time a population of non-dividing cells remains viable when deprived of nutrients. The life span of cells can be increased by at least about 20%, 30%, 40%, 50%, 60% or between 20% and 70%, 30% and 60%, 40 and 60% or more using the methods of the invention.

"Sir2 family members" or "Sir2 protein family members" refers to *S. cerevisiae* Sir2 protein as well as any histone deacetylases having substantial structural similarities to Sir2, e.g., the human homologs hSIRT1, hSIRT2, hSIRT3, hSIRT4, hSIRT5, hSIRT6 and hSIRT7; and Sir-2.1.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays described herein.

The term "specific hybridization" of a probe to a target site of a template nucleic acid refers to hybridization of the probe predominantly to the target, such that the hybridization signal can be clearly interpreted. As further described herein, such conditions resulting in specific hybridization vary depending on the length of the region of homology, the GC content of the region, the melting temperature "Tm" of the hybrid. Hybridization conditions will thus vary in the salt content, acidity, and temperature of the hybridization solution and the washes.

"Stress" refers to any non-optimal condition for growth, development or reproduction. A "stress condition" can be exposure to heatshock; osmotic stress; a DNA damaging agent; inadequate salt level; inadequate nitrogen levels; inadequate nutrient level; radiation or a toxic compound, e.g., a toxin or chemical warfare agent (such as dirty bombs and other weapons that may be used in bioterrorism). "Inadequate levels" refer to levels that result in non-optimal condition for growth, development or reproduction.

"Treating" a condition or disease refers to curing as well as ameliorating at least one symptom of the condition or disease.

The term "therapeutic agent" is art-recognized and refers to any chemical moiety that is a biologically, physiologically, or pharmacologically active substance that acts locally or systemically in a subject. The term also means any substance intended for use in the diagnosis, cure, mitigation, treatment or prevention of disease or in the enhancement of desirable physical or mental development and/or conditions in an animal or human.

The term "therapeutic effect" is art-recognized and refers to a local or systemic effect in animals, particularly mammals, and more particularly humans caused by a pharmacologically active substance. The phrase "therapeutically-effective amount" means that amount of such a substance that produces some desired local or systemic effect at a reasonable benefit/risk ratio applicable to any treatment. The therapeutically effective amount of such substance will vary depending upon the subject and disease or condition being treated, the weight and age of the subject, the severity of the disease or condition, the manner of administration and the like, which can readily be determined by one of ordinary skill in the art. For example, certain compositions described herein may be administered in a sufficient amount to produce a desired effect at a reasonable benefit/risk ratio applicable to such treatment.

A "variant" of a polypeptide refers to a polypeptide having the amino acid sequence of the polypeptide in which is altered in one or more amino acid residues. The variant may have "conservative" changes, wherein a substituted amino acid has similar structural or chemical properties (e.g., replacement of leucine with isoleucine). A variant may have "nonconservative" changes (e.g., replacement of glycine with tryptophan). Analogous minor variations may also include amino acid deletions or insertions, or both. Guidance in determining which amino acid residues may be substituted, inserted, or deleted without abolishing biological or immunological activity may be found using computer programs well known in the art, for example, LASERGENE software (DNASTAR).

The term "variant," when used in the context of a polynucleotide sequence, may encompass a polynucleotide sequence related to that of a particular gene or the coding sequence thereof. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. The resulting polypeptides generally will have significant amino acid identity relative to each other. A polymorphic variation is a variation in the polynucleotide sequence of a particular gene between individuals of a given species. Polymorphic variants also may encompass "single nucleotide polymorphisms" (SNPs) in which the polynucleotide sequence varies by one base. The presence of SNPs may be indicative of, for example, a certain population, a disease state, or a propensity for a disease state.

The term "aliphatic" is art-recognized and refers to a linear, branched, cyclic alkane, alkene, or alkyne. In certain embodiments, aliphatic groups in the present invention are linear or branched and have from 1 to about 20 carbon atoms.

The term "alkyl" is art-recognized, and includes saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight chain or branched chain alkyl has about 30 or fewer carbon atoms in its backbone (e.g., C₁-C₃₀ for straight chain, C₃-C₃₀ for branched chain), and alternatively, about 20 or fewer. Likewise, cycloalkyls have from about 3 to about 10 carbon atoms in their ring structure, and alternatively about 5, 6 or 7 carbons in the ring structure. The term "alkyl" is also defined to include halosubstituted alkyls.

The term "aralkyl" is art-recognized and refers to an alkyl group substituted with an aryl group (e.g., an aromatic or heteroaromatic group).

The terms "alkenyl" and "alkynyl" are art-recognized and refer to unsaturated aliphatic groups analogous in length and possible substitution to the alkyls described above, but that contain at least one double or triple bond respectively.

Unless the number of carbons is otherwise specified, "lower alkyl" refers to an alkyl group, as defined above, but having from one to about ten carbons, alternatively from one to about six carbon atoms in its backbone structure. Likewise, "lower alkenyl" and "lower alkynyl" have similar chain lengths.

The term "heteroatom" is art-recognized and refers to an atom of any element other than carbon or hydrogen. Illustrative heteroatoms include boron, nitrogen, oxygen, phosphorus, sulfur and selenium.

The term "aryl" is art-recognized and refers to 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring may be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings may be cycloalkyls, cycloalkenyls, cycloalkynyls, aryls and/or heterocyclyls.

The terms ortho, meta and para are art-recognized and refer to 1,2-, 1,3- and 1,4-disubstituted benzenes, respectively. For example, the names 1,2-dimethylbenzene and ortho-dimethylbenzene are synonymous.

The terms "heterocyclyl" or "heterocyclic group" are art-recognized and refer to 3-to about 10-membered ring structures, alternatively 3- to about 7-membered rings, whose ring structures include one to four heteroatoms. Heterocycles may also be polycycles. Heterocyclyl groups include, for example, thiophene, thianthrene, furan, pyran, isobenzofuran, chromene, xanthene, phenoxanthene, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring may be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The terms "polycyclyl" or "polycyclic group" are art-recognized and refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle may be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "carbocycle" is art-recognized and refers to an aromatic or non-aromatic ring in which each atom of the ring is carbon.

The term "nitro" is art-recognized and refers to -NO₂; the term "halogen" is art-recognized and refers to -F, -Cl, -Br or -I; the term "sulfhydryl" is art-recognized and refers to -SH; the term "hydroxyl" means -OH; and the term "sulfonyl" is art-recognized and refers to -SO₂⁻. "Halide" designates the corresponding anion of the halogens, and "pseudohalide" has the definition set forth on 560 of "Advanced Inorganic Chemistry" by Cotton and Wilkinson.

The terms "amine" and "amino" are art-recognized and refer to both unsubstituted and substituted amines, e.g., a moiety that may be represented by the general formulas: 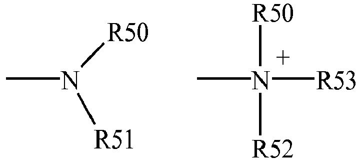 wherein R50, R51 and R52 each independently represent a hydrogen, an alkyl, an alkenyl, - (CH₂)ₘ-R61, or R50 and R51, taken together with the N atom to which they are attached complete a heterocycle having from 4 to 8 atoms in the ring structure; R61 represents an aryl, a cycloalkyl, a cycloalkenyl, a heterocycle or a polycycle; and m is zero or an integer in the range of 1 to 8. In certain embodiments, only one of R50 or R51 may be a carbonyl, e.g., R50, R51 and the nitrogen together do not form an imide. In other embodiments, R50 and R51 (and optionally R52) each independently represent a hydrogen, an alkyl, an alkenyl, or -(CH₂)ₘ-R61. Thus, the term "alkylamine" includes an amine group, as defined above, having a substituted or unsubstituted alkyl attached thereto, i.e., at least one of R50 and R51 is an alkyl group.

The term "acylamino" is art-recognized and refers to a moiety that may be represented by the general formula: 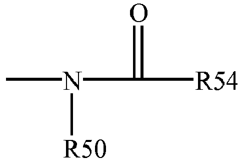 wherein R50 is as defined above, and R54 represents a hydrogen, an alkyl, an alkenyl or - (CH₂)ₘ-R61, where m and R61 are as defined above.

The term "amido" is art recognized as an amino-substituted carbonyl and includes a moiety that may be represented by the general formula: 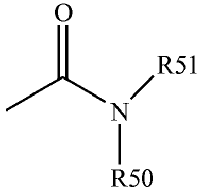 wherein R50 and R51 are as defined above. Certain embodiments of the amide in the present invention will not include imides which may be unstable.

The term "alkylthio" refers to an alkyl group, as defined above, having a sulfur radical attached thereto. In certain embodiments, the "alkylthio" moiety is represented by one of -S-alkyl, -S-alkenyl, -S-alkynyl, and -S-(CH₂)ₘ-R61, wherein m and R61 are defined above. Representative alkylthio groups include methylthio, ethyl thio, and the like.

The term "carbonyl" is art recognized and includes such moieties as may be represented by the general formulas: 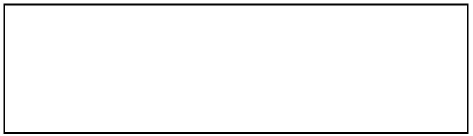 wherein X50 is a bond or represents an oxygen or a sulfur, and R55 and R56 represents a hydrogen, an alkyl, an alkenyl, -(CH₂)ₘ-R61or a pharmaceutically acceptable salt, R56 represents a hydrogen, an alkyl, an alkenyl or -(CH₂)ₘ-R61, where m and R61 are defined above. Where X50 is an oxygen and R55 or R56 is not hydrogen, the formula represents an "ester". Where X50 is an oxygen, and R55 is as defined above, the moiety is referred to herein as a carboxyl group, and particularly when R55 is a hydrogen, the formula represents a "carboxylic acid". Where X50 is an oxygen, and R56 is hydrogen, the formula represents a "formate". In general, where the oxygen atom of the above formula is replaced by sulfur, the formula represents a "thiolcarbonyl" group. Where X50 is a sulfur and R55 or R56 is not hydrogen, the formula represents a "thiolester." Where X50 is a sulfur and R55 is hydrogen, the formula represents a "thiolcarboxylic acid." Where X50 is a sulfur and R56 is hydrogen, the formula represents a "thiolformate." On the other hand, where X50 is a bond, and R55 is not hydrogen, the above formula represents a "ketone" group. Where X50 is a bond, and R55 is hydrogen, the above formula represents an "aldehyde" group.

The terms "alkoxyl" or "alkoxy" are art-recognized and refer to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as may be represented by one of -O-alkyl, -O-alkenyl, -O-alkynyl, -O--(CH₂)ₘ-R61, where m and R61 are described above.

The definition of each expression, e.g. alkyl, m, n, and the like, when it occurs more than once in any structure, is intended to be independent of its definition elsewhere in the same structure.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, methanesulfonate ester, and nonafluorobutanesulfonate ester functional groups and molecules that contain said groups, respectively.

The abbreviations Me, Et, Ph, Tf, Nf, Ts, and Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by organic chemists of ordinary skill in the art appears in the first issue of each volume of the Journal of Organic Chemistry; this list is typically presented in a table entitled Standard List of Abbreviations.

Certain compounds contained in compositions of the present invention may exist in particular geometric or stereoisomeric forms. In addition, polymers of the present invention may also be optically active. The present invention contemplates all such compounds, including cis- and trans-isomers, *R*- and *S*-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds that may be substituted or unsubstituted.

The definition of each expression, e.g. lower alkyl, m, n, p and the like, when it occurs more than once in any structure, is intended to be independent of its definition elsewhere in the same structure.

The term "pharmaceutically-acceptable salts" is art-recognized and refers to the relatively non-toxic, inorganic and organic acid addition salts of compounds, including, for example, those contained in compositions of the present invention.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof from one organ, or portion of the body, to another organ, or portion of the body. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The terms "systemic administration," "administered systemically," "peripheral administration" and "administered peripherally" are art-recognized and refer to the administration of a subject composition, therapeutic or other material other than directly into the central nervous system, such that it enters the patient's system and, thus, is subject to metabolism and other like processes, for example, subcutaneous administration.

The terms "parenteral administration" and "administered parenterally" are art-recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articulare, subcapsular, subarachnoid, intraspinal, and intrasternal injection and infusion.

### Exemplary Compounds of the Invention

In certain embodiments, the invention relates to a compound of formula **I** or a compound of formula **II**: 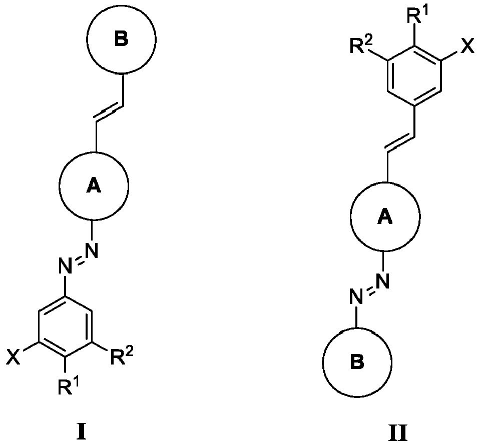 wherein 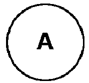
is an aryl heterocycle diradical; 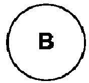
is heteroaryl;
X is halo;
R¹ is hydroxy, alkoxy, or amino; and
R² is hydroxy, alkoxy, or amino.

In certain embodiments, the invention relates to any one of the aforementioned compounds, wherein 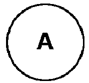 is a diradical of azaindole, benzo(b)thiene, benzimidazole, benzofuran, benzoxazole, benzothiazole, benzothiadiazole, benzotriazole, benzoxadiazole, furan, imidazole, imidazopyridine, indole, indoline, indazole, isoindoline, isoxazole, isothiazole, isoquinoline, oxadiazole, oxazole, purine, pyran, pyrazine, pyrazole, pyridine, pyrimidine, pyrrole, pyrrolo[2,3-d]pyrimidine, pyrazolo[3,4-d]pyrimidine, quinoline, quinazoline, triazole, thiazole, thiobenzene, tetrahydroindole, tetrazole, thiadiazole, thiophene, thiomorpholine, or triazole.

In certain embodiments, the invention relates to any one of the aforementioned compounds, wherein 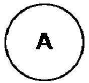 is a diradical of furan, imidazole, isoxazole, isothiazole, oxadiazole, oxazole, pyrrole, triazole, thiazole, tetrazole, thiadiazole, thiophene, or triazole.

In certain embodiments, the invention relates to any one of the aforementioned compounds, wherein 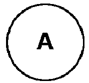 is a diradical of imidazole.

In certain embodiments, the invention relates to any one of the aforementioned compounds, wherein 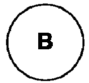 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned compounds, wherein 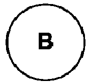 is furanyl, imidazolyl, isoxazolyl, isothiazolyl, oxadiazolyl, oxazolyl, pyrrolyl, triazolyl, thiazolyl, tetrazolyl, thiadiazolyl, thienyl, or triazolyl.

In certain embodiments, the invention relates to any one of the aforementioned compounds, wherein 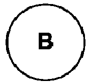 is imidazolyl.

In certain embodiments, the invention relates to any one of the aforementioned compounds, wherein X is bromo.

In certain embodiments, the invention relates to any one of the aforementioned compounds, wherein R¹ is hydroxy.

In certain embodiments, the invention relates to any one of the aforementioned compounds, wherein R² is alkoxy.

In certain embodiments, the invention relates to any one of the aforementioned compounds, wherein R² is methoxy.

In certain embodiments, the invention relates to a compound of formula **III**: 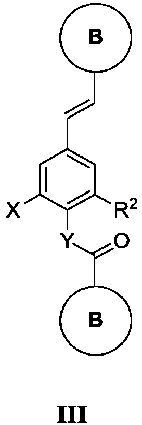 wherein, independently for each occurrence, 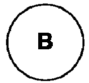
is heteroaryl;
X is halo;
R² is hydroxy, alkoxy, or amino; and
Y is -O- or -NH-.

In certain embodiments, the invention relates to any one of the aforementioned compounds, wherein 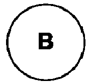 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned compounds, wherein 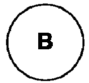 is furanyl, imidazolyl, isoxazolyl, isothiazolyl, oxadiazolyl, oxazolyl, pyrrolyl, triazolyl, thiazolyl, tetrazolyl, thiadiazolyl, thienyl, or triazolyl.

In certain embodiments, the invention relates to any one of the aforementioned compounds, wherein 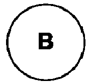 is imidazolyl.

In certain embodiments, the invention relates to any one of the aforementioned compounds, wherein X is bromo.

In certain embodiments, the invention relates to any one of the aforementioned compounds, wherein R² is alkoxy.

In certain embodiments, the invention relates to any one of the aforementioned compounds, wherein R² is methoxy.

In certain embodiments, the invention relates to any one of the aforementioned compounds, wherein Y is -O-.

In certain embodiments, the invention relates to a compound of formula **IV**: 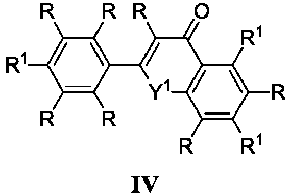 wherein, independently for each occurrence,
R is -H, halo, aryl, nitro, alkyl, hydroxy, alkoxy, or amino;
R¹ is hydroxy, alkoxy, or amino; and
Y¹ is -S-, -O-, or -NH-.

In certain embodiments, the invention relates to any one of the aforementioned compounds, wherein one instance of R is hydroxy. In certain embodiments, the invention relates to any one of the aforementioned compounds, wherein one instance of R is hydroxy; and the remaining instances of R are -H.

In certain embodiments, the invention relates to any one of the aforementioned compounds, wherein R¹ is hydroxy.

In certain embodiments, the invention relates to any one of the aforementioned compounds, wherein Y¹ is -O-.

In certain embodiments, the invention relates to any one of the aforementioned compounds, provided the compound is not

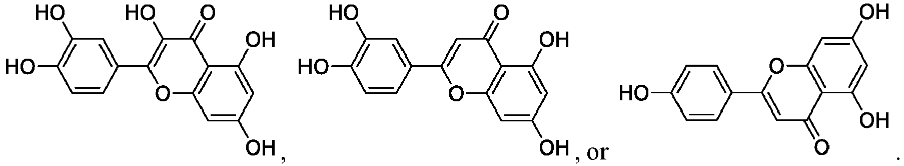

In certain embodiments, the invention relates to a compound of formula **V**: 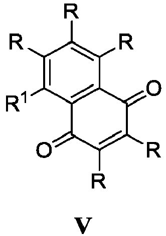 wherein, independently for each occurrence,
R is -H, halo, aryl, nitro, alkyl, hydroxy, alkoxy, or amino; and
R¹ is hydroxy, alkoxy, or amino.

In certain embodiments, the invention relates to any one of the aforementioned compounds, wherein R is -H.

In certain embodiments, the invention relates to any one of the aforementioned compounds, wherein R¹ is hydroxy.

In certain embodiments, the invention relates to any one of the aforementioned compounds, provided the compound is not

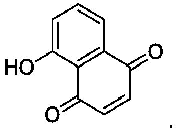

In certain embodiments, the invention relates to a compound of formula **VI**: 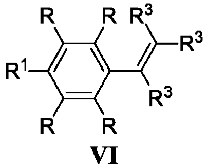 wherein, independently for each occurrence,
R is -H, halo, aryl, nitro, alkyl, hydroxy, alkoxy, or amino;
R¹ is hydroxy, alkoxy, or amino;
R³ is -H, cyano, -CO₂R⁴, or -C(O)N(R⁴)₂; and
R⁴ is -H or alkyl.

In certain embodiments, the invention relates to any one of the aforementioned compounds, wherein R is -H.

In certain embodiments, the invention relates to any one of the aforementioned compounds, wherein R¹ is hydroxy.

In certain embodiments, the invention relates to any one of the aforementioned compounds, wherein at least one instance of R³ is cyano. In certain embodiments, the invention relates to any one of the aforementioned compounds, wherein at least two instances of R³ are cyano.

In certain embodiments, the invention relates to any one of the aforementioned compounds, provided the compound is not

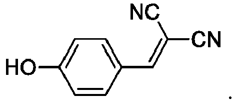

In certain embodiments, the invention relates to a compound of formula **VII**: 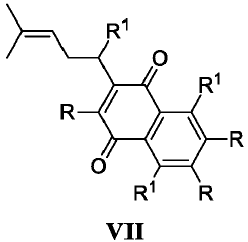 wherein, independently for each occurrence,
R is -H, halo, aryl, nitro, alkyl, hydroxy, alkoxy, or amino; and
R¹ is hydroxy, alkoxy, or amino.

In certain embodiments, the invention relates to any one of the aforementioned compounds, wherein R is -H.

In certain embodiments, the invention relates to any one of the aforementioned compounds, wherein R¹ is hydroxy.

In certain embodiments, the invention relates to any one of the aforementioned compounds, provided the compound is not

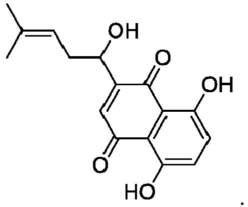

In certain embodiments, the invention relates to a compound of formula **VIII**: 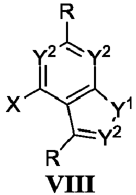 wherein, independently for each occurrence,
R is -H, halo, aryl, nitro, alkyl, hydroxy, alkoxy, or amino;
X is halo;
Y¹ is -O-, -S-, or -NH-; and
Y² is =N- or =CR-.

In certain embodiments, the invention relates to any one of the aforementioned compounds, wherein R is -H.

In certain embodiments, the invention relates to any one of the aforementioned compounds, wherein X is chloro.

In certain embodiments, the invention relates to any one of the aforementioned compounds, wherein Y¹ is -NH-.

In certain embodiments, the invention relates to any one of the aforementioned compounds, wherein Y² is =N-.

In certain embodiments, the invention relates to any one of the aforementioned compounds, provided the compound is not

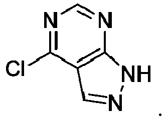

In certain embodiments, the invention relates to a compound of formula **IX**: 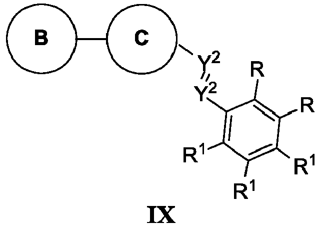 wherein, independently for each occurrence, 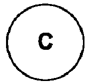
is a five-membered, unsaturated heterocycle diradical; 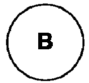
is heteroaryl;
R is -H, halo, aryl, nitro, alkyl, hydroxy, alkoxy, or amino;
R¹ is hydroxy, alkoxy, or amino; and
Y² is =N- or =CR-.

In certain embodiments, the invention relates to any one of the aforementioned compounds, wherein 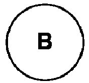 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned compounds, wherein R is -H.

In certain embodiments, the invention relates to any one of the aforementioned compounds, wherein R¹ is hydroxy.

In certain embodiments, the invention relates to any one of the aforementioned compounds, wherein one instance of Y² is =N-.

In certain embodiments, the invention relates to any one of the aforementioned compounds, provided the compound is not

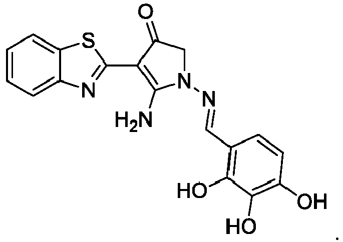

In certain embodiments, the invention relates to a compound of formula **X**: 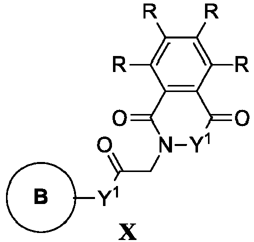 wherein, independently for each occurrence, 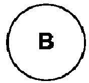
is heteroaryl;
R is -H, halo, aryl, nitro, alkyl, hydroxy, alkoxy, or amino;
Y¹ is -S-, -O-, or -NH-.

In certain embodiments, the invention relates to any one of the aforementioned compounds, wherein 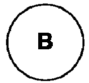 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned compounds, wherein R is -H.

In certain embodiments, the invention relates to any one of the aforementioned compounds, wherein Y¹ is -NH-.

In certain embodiments, the invention relates to any one of the aforementioned compounds, provided the compound is not

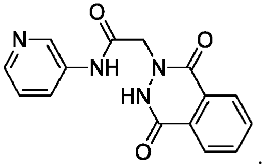

In certain embodiments, the invention relates to a compound of formula **XI**: 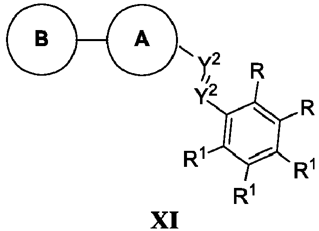 wherein, independently for each occurrence, 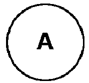
is an aryl heterocycle diradical; 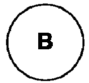
is heteroaryl;
R is -H, halo, aryl, nitro, alkyl, hydroxy, alkoxy, or amino;
R¹ is hydroxy, alkoxy, or amino; and
Y² is =N- or =CR-.

In certain embodiments, the invention relates to any one of the aforementioned compounds, wherein 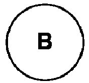 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned compounds, wherein R is -H.

In certain embodiments, the invention relates to any one of the aforementioned compounds, wherein R¹ is hydroxy.

In certain embodiments, the invention relates to any one of the aforementioned compounds, wherein one instance of Y² is =N-.

In certain embodiments, the invention relates to any one of the aforementioned compounds, provided the compound is not

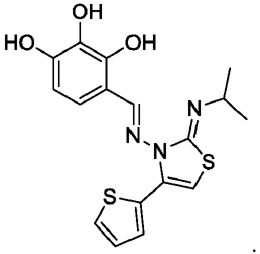

In certain embodiments, the invention relates to a compound of formula **XII**: 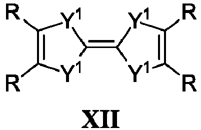 wherein, independently for each occurrence,
R is -H, halo, aryl, nitro, alkyl, hydroxy, alkoxy, or amino; and
Y¹ is -S-, -O-, or -NH-.

In certain embodiments, the invention relates to any one of the aforementioned compounds, wherein at least one instance of R is -H.

In certain embodiments, the invention relates to any one of the aforementioned compounds, wherein at least one instance of Y¹ is -S-.

In certain embodiments, the invention relates to any one of the aforementioned compounds, provided the compound is not

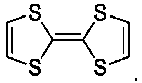

In certain embodiments, the invention relates to a compound of formula **XIII**: 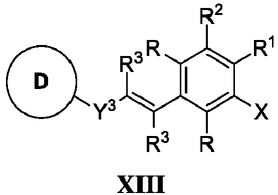 wherein, independently for each occurrence, 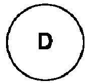
is aryl or heteroaryl;
R is -H, halo, aryl, nitro, alkyl, hydroxy, alkoxy, or amino;
R¹ is hydroxy, alkoxy, or amino;
R² is hydroxy, alkoxy, or amino;
R³ is -H, cyano, -CO₂R⁴, or -C(O)N(R⁴)₂;
X is halo;
Y³ is a bond, -C(O)-d, -C(O)NH-d, -NH-C(O)-d, or -C(O)NH-CH₂-d; and
d is a bond to 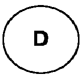 .

In certain embodiments, the invention relates to any one of the aforementioned compounds, wherein R is -H

In certain embodiments, the invention relates to any one of the aforementioned compounds, wherein R¹ is hydroxy.

In certain embodiments, the invention relates to any one of the aforementioned compounds, wherein R² is alkoxy. In certain embodiments, the invention relates to any one of the aforementioned compounds, wherein R² is methoxy.

In certain embodiments, the invention relates to any one of the aforementioned compounds, wherein one instance of R³ is cyano.

In certain embodiments, the invention relates to any one of the aforementioned compounds, wherein R³ is -H.

In certain embodiments, the invention relates to any one of the aforementioned compounds, wherein X is bromo.

In certain embodiments, the invention relates to any one of the aforementioned compounds, wherein Y³ is a bond. In certain embodiments, the invention relates to any one of the aforementioned compounds, wherein Y³ is -C(O)-d. In certain embodiments, the invention relates to any one of the aforementioned compounds, wherein Y³ is -C(O)NH-d. In certain embodiments, the invention relates to any one of the aforementioned compounds, wherein Y³ is -NH-C(O)-d. In certain embodiments, the invention relates to any one of the aforementioned compounds, wherein Y³ is -C(O)NH-CH₂-d.

In certain embodiments, the invention relates to any one of the aforementioned compounds, provided the compound is not 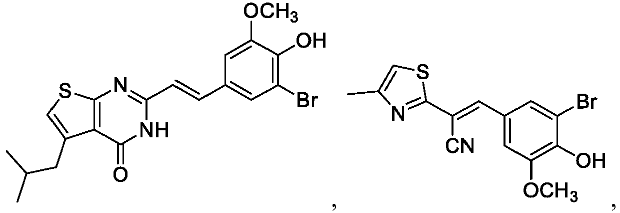 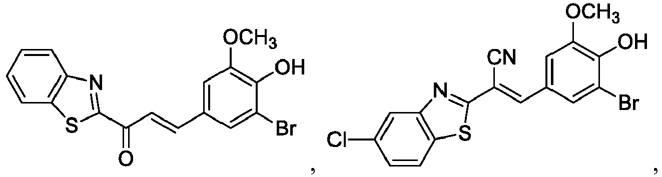 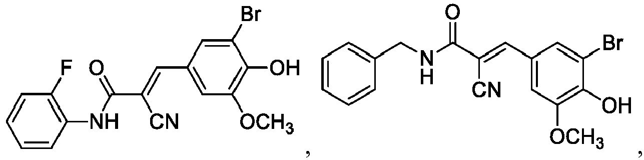 or

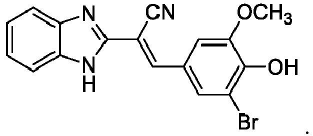

In certain embodiments, the invention relates to a compound of formula **XIV**: 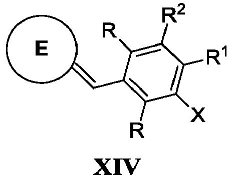 wherein, independently for each occurrence, 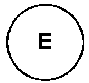
is a five-membered heterocycle radical;
R is -H, halo, aryl, nitro, alkyl, hydroxy, alkoxy, or amino;
R¹ is hydroxy, alkoxy, or amino;
R² is hydroxy, alkoxy, or amino; and
X is halo.

In certain embodiments, the invention relates to any one of the aforementioned compounds, wherein R is -H

In certain embodiments, the invention relates to any one of the aforementioned compounds, wherein R¹ is hydroxy.

In certain embodiments, the invention relates to any one of the aforementioned compounds, wherein R² is alkoxy. In certain embodiments, the invention relates to any one of the aforementioned compounds, wherein R² is methoxy.

In certain embodiments, the invention relates to any one of the aforementioned compounds, wherein X is bromo.

In certain embodiments, the invention relates to any one of the aforementioned compounds, provided the compound is not

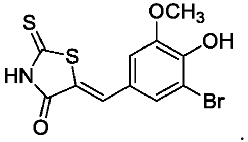

In certain embodiments, the invention relates to a compound of formula **XV**: 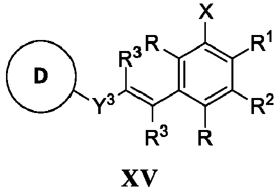 wherein, independently for each occurrence, 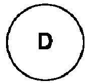
is aryl or heteroaryl;
R is -H, halo, aryl, nitro, alkyl, hydroxy, alkoxy, or amino;
R¹ is hydroxy, alkoxy, or amino;
R² is hydroxy, alkoxy, or amino;
R³ is -H, cyano, -CO₂R⁴, or -C(O)N(R⁴)₂;
X is halo;
Y³ is a bond, -C(O)-d, -C(O)NH-d, -NH-C(O)-d, or -C(O)NH-CH₂-d; and
d is a bond to

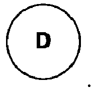

In certain embodiments, the invention relates to any one of the aforementioned compounds, wherein R is -H

In certain embodiments, the invention relates to any one of the aforementioned compounds, wherein R¹ is hydroxy.

In certain embodiments, the invention relates to any one of the aforementioned compounds, wherein R² is alkoxy. In certain embodiments, the invention relates to any one of the aforementioned compounds, wherein R² is methoxy.

In certain embodiments, the invention relates to any one of the aforementioned compounds, wherein one instance of R³ is cyano.

In certain embodiments, the invention relates to any one of the aforementioned compounds, wherein R³ is -H.

In certain embodiments, the invention relates to any one of the aforementioned compounds, wherein X is bromo.

In certain embodiments, the invention relates to any one of the aforementioned compounds, wherein Y³ is a bond. In certain embodiments, the invention relates to any one of the aforementioned compounds, wherein Y³ is -C(O)-d. In certain embodiments, the invention relates to any one of the aforementioned compounds, wherein Y³ is -C(O)NH-d. In certain embodiments, the invention relates to any one of the aforementioned compounds, wherein Y³ is -NH-C(O)-d. In certain embodiments, the invention relates to any one of the aforementioned compounds, wherein Y³ is -C(O)NH-CH₂-d.

In certain embodiments, the invention relates to any one of the aforementioned compounds, provided the compound is not 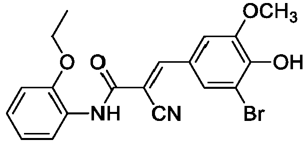 or

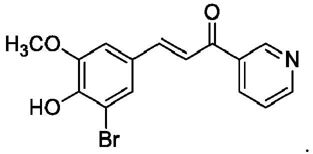

### Exemplary Methods of the Invention

In certain embodiments, the invention relates to a method of inhibiting CD38 in a cell, comprising
contacting the cell with a therapeutically effective amount of an agent.

In certain embodiments, the invention relates to a method of inhibiting NADase activity in a cell, comprising
contacting the cell with a therapeutically effective amount of an agent.

In certain embodiments, the invention relates to a method of increasing NAD levels in a cell, comprising
contacting the cell with a therapeutically effective amount of an agent.

In certain embodiments, the invention relates to the aforementioned method of increasing NAD levels in a cell, thereby increasing mito biogenesis.

In certain embodiments, the invention relates to a method of increasing NAD⁺ levels in a cell, comprising
contacting the cell with a therapeutically effective amount of an agent.

In certain embodiments, the invention relates to a method of activating a PARP in a cell, comprising
contacting the cell with a therapeutically effective amount of an agent.

In certain embodiments, the invention relates to any one of the aforementioned methods, wherein multiple PARPs are activated.

In certain embodiments, the invention relates to a method of treating a subject, comprising
administering to a subject in need thereof a therapeutically effective amount of an agent.

In certain embodiments, the invention relates to a method of retarding, treating, or preventing an age-related disease in a subject, comprising
administering to a subject in need thereof a therapeutically effective amount of an agent.

In certain embodiments, the invention relates to a method of retarding aging in a subject, comprising
administering to a subject in need thereof a therapeutically effective amount of an agent.

In certain embodiments, the invention relates to a method of imparting the benefits of diet and exercise to a subject, comprising
administering to a subject in need thereof a therapeutically effective amount of an agent.

In certain embodiments, the invention relates to any one of the aforementioned methods, wherein the age-related disease is selected from the group consisting of neurodegeneration, dementia, a metabolic disease, osteoporosis, an inflammatory disorder, cataracts, bone loss, cardiac dysfunction, cardiovascular diseases, cancer, and a disease resulting from mitochondrial dysfunction.

In certain embodiments, the invention relates to any one of the aforementioned methods, wherein the metabolic disease is selected from the group consisting of type 2 diabetes, shortness of breath, gallbladder disease, hypertension, elevated blood cholesterol levels, cancer, osteoarthritis, other orthopedic problems, reflux esophagitis, snoring, heart trouble, dyslipidemia, coronary heart disease, stroke, hyperinsulinemia, depression, anxiety, gout, fatty liver disease, insulin resistance, pre-diabetes, hypercoagulation, sepsis, inflammatory bowel diseases, dementia, beta-cell dysfunction, sleep apnea, obstructive sleep apnea, hypopnea, and visceral adiposity.

In certain embodiments, the invention relates to any one of the aforementioned methods, wherein the subject is likely to develop obesity (e.g., mammals having an elevated risk of developing diet-induced obesity). A mammal can be identified as having or being likely to develop obesity using standard clinical techniques. For example, analysis of a human's family history or eating habits can be used to determine whether or not the human is likely to develop an obesity condition. As described herein, a mammal identified as having or being susceptible to developing an obesity condition can be treated by administering an agent, e.g., an inhibitor of CD38.

In certain embodiments, the invention relates to any one of the aforementioned methods, wherein the cancer is selected from the group consisting of endometrial, breast, prostate, and colon.

In certain embodiments, a variety of other disorders may be retarded, treated, or prevented according to the inventive methods. These include, but are not limited to, diabetes, gestational diabetes, obesity, metabolic syndrome, insulin-resistance syndromes, syndrome X, insulin resistance, high blood pressure, hypertension, high blood cholesterol, dyslipidemia, hyperlipidemia, dyslipidemia, atherosclerotic disease including stroke, coronary artery disease or myocardial infarction, hyperglycemia, hyperinsulinemia and/or hyperproinsulinemia, impaired glucose tolerance, delayed insulin release, diabetic complications, including coronary heart disease, angina pectoris, congestive heart failure, stroke, cognitive functions in dementia, retinopathy, peripheral neuropathy, nephropathy, glomerulonephritis, glomerulosclerosis, nephrotic syndrome, hypertensive nephrosclerosis some types of cancer (such as endometrial, breast, prostate, and colon), lipodystrophy, cholesterol related disorders, such as gallstones, cholescystitis and cholelithiasis, gout, obstructive sleep apnea and respiratory problems, osteoarthritis, and prevention and treatment of bone loss, e.g. osteoporosis.

In certain embodiments, the invention relates to any one of the aforementioned methods, wherein the agent is a compound of formula **I** or a compound of formula **II**: 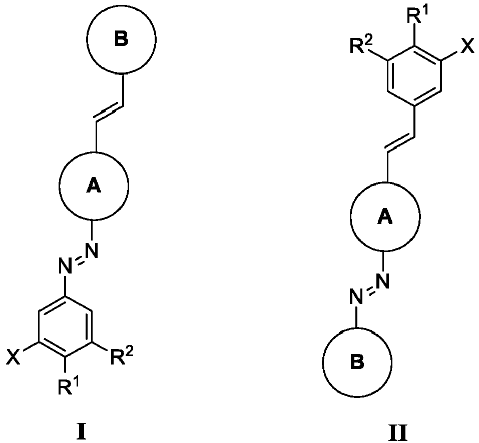 wherein 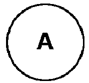
is an aryl heterocycle diradical; 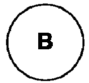
is heteroaryl;
X is halo;
R¹ is hydroxy, alkoxy, or amino; and
R² is hydroxy, alkoxy, or amino.

In certain embodiments, the invention relates to any one of the aforementioned methods, wherein 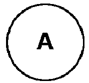 is a diradical of azaindole, benzo(b)thiene, benzimidazole, benzofuran, benzoxazole, benzothiazole, benzothiadiazole, benzotriazole, benzoxadiazole, furan, imidazole, imidazopyridine, indole, indoline, indazole, isoindoline, isoxazole, isothiazole, isoquinoline, oxadiazole, oxazole, purine, pyran, pyrazine, pyrazole, pyridine, pyrimidine, pyrrole, pyrrolo[2,3-d]pyrimidine, pyrazolo[3,4-d]pyrimidine, quinoline, quinazoline, triazole, thiazole, thiobenzene, tetrahydroindole, tetrazole, thiadiazole, thiophene, thiomorpholine, or triazole.

In certain embodiments, the invention relates to any one of the aforementioned methods, wherein 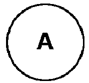 is a diradical of furan, imidazole, isoxazole, isothiazole, oxadiazole, oxazole, pyrrole, triazole, thiazole, tetrazole, thiadiazole, thiophene, or triazole.

In certain embodiments, the invention relates to any one of the aforementioned methods, wherein 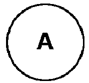 is a diradical of imidazole.

In certain embodiments, the invention relates to any one of the aforementioned methods, wherein 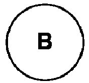 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned methods, wherein 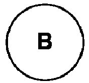 is furanyl, imidazolyl, isoxazolyl, isothiazolyl, oxadiazolyl, oxazolyl, pyrrolyl, triazolyl, thiazolyl, tetrazolyl, thiadiazolyl, thienyl, or triazolyl.

In certain embodiments, the invention relates to any one of the aforementioned methods, wherein 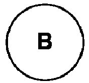 is imidazolyl.

In certain embodiments, the invention relates to any one of the aforementioned methods, wherein X is bromo.

In certain embodiments, the invention relates to any one of the aforementioned methods, wherein R¹ is hydroxy.

In certain embodiments, the invention relates to any one of the aforementioned methods, wherein R² is alkoxy.

In certain embodiments, the invention relates to any one of the aforementioned methods, wherein R² is methoxy.

In certain embodiments, the invention relates to any one of the aforementioned methods, wherein the agent is a compound of formula **III**: 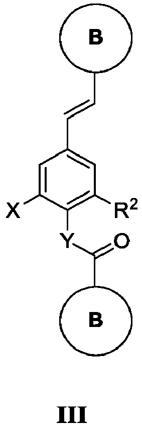 wherein, independently for each occurrence, 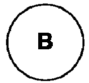
is heteroaryl;
X is halo;
R² is hydroxy, alkoxy, or amino; and
Y is -O- or -NH-.

In certain embodiments, the invention relates to any one of the aforementioned methods, wherein 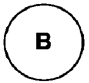 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned methods, wherein 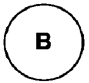 is furanyl, imidazolyl, isoxazolyl, isothiazolyl, oxadiazolyl, oxazolyl, pyrrolyl, triazolyl, thiazolyl, tetrazolyl, thiadiazolyl, thienyl, or triazolyl.

In certain embodiments, the invention relates to any one of the aforementioned methods, wherein 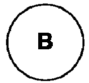 is imidazolyl.

In certain embodiments, the invention relates to any one of the aforementioned methods, wherein X is bromo.

In certain embodiments, the invention relates to any one of the aforementioned methods, wherein R² is alkoxy.

In certain embodiments, the invention relates to any one of the aforementioned methods, wherein R² is methoxy.

In certain embodiments, the invention relates to any one of the aforementioned methods, wherein Y is -O-.

In certain embodiments, the invention relates to any one of the aforementioned methods, wherein the agent is a compound of formula **IV**: 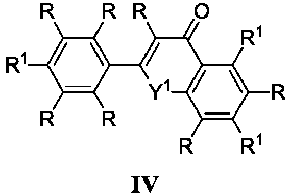 wherein, independently for each occurrence,
R is -H, halo, aryl, nitro, alkyl, hydroxy, alkoxy, or amino;
R¹ is hydroxy, alkoxy, or amino; and
Y¹ is -S-, -O-, or -NH-.

In certain embodiments, the invention relates to any one of the aforementioned methods, wherein one instance of R is hydroxy. In certain embodiments, the invention relates to any one of the aforementioned methods, wherein one instance of R is hydroxy; and the remaining instances of R are -H.

In certain embodiments, the invention relates to any one of the aforementioned methods, wherein R¹ is hydroxy.

In certain embodiments, the invention relates to any one of the aforementioned methods, wherein Y¹ is -O-.

In certain embodiments, the invention relates to any one of the aforementioned methods, wherein the agent is a compound of formula **V**: 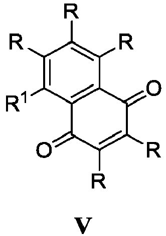 wherein, independently for each occurrence,
R is -H, halo, aryl, nitro, alkyl, hydroxy, alkoxy, or amino; and
R¹ is hydroxy, alkoxy, or amino.

In certain embodiments, the invention relates to any one of the aforementioned methods, wherein R is -H.

In certain embodiments, the invention relates to any one of the aforementioned methods, wherein R¹ is hydroxy.

In certain embodiments, the invention relates to any one of the aforementioned methods, wherein the agent is a compound of formula **VI**: 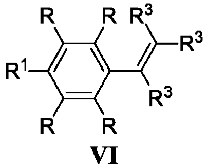 wherein, independently for each occurrence,
R is -H, halo, aryl, nitro, alkyl, hydroxy, alkoxy, or amino;
R¹ is hydroxy, alkoxy, or amino;
R³ is -H, cyano, -CO₂R⁴, or -C(O)N(R⁴)₂; and
R⁴ is -H or alkyl.

In certain embodiments, the invention relates to any one of the aforementioned methods, wherein R is -H.

In certain embodiments, the invention relates to any one of the aforementioned methods, wherein R¹ is hydroxy.

In certain embodiments, the invention relates to any one of the aforementioned methods, wherein at least one instance of R³ is cyano. In certain embodiments, the invention relates to any one of the aforementioned methods, wherein at least two instances of R³ are cyano.

In certain embodiments, the invention relates to any one of the aforementioned methods, wherein the agent is a compound of formula **VII**: 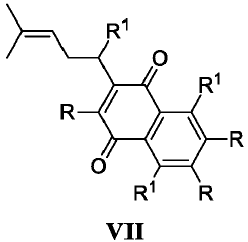 wherein, independently for each occurrence,
R is -H, halo, aryl, nitro, alkyl, hydroxy, alkoxy, or amino; and
R¹ is hydroxy, alkoxy, or amino.

In certain embodiments, the invention relates to any one of the aforementioned methods, wherein R is -H.

In certain embodiments, the invention relates to any one of the aforementioned methods, wherein R¹ is hydroxy.

In certain embodiments, the invention relates to any one of the aforementioned methods, wherein the agent is compound of formula **VIII**: 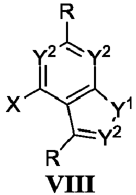 wherein, independently for each occurrence,
R is -H, halo, aryl, nitro, alkyl, hydroxy, alkoxy, or amino;
X is halo;
Y¹ is -O-, -S-, or -NH-; and
Y² is =N- or =CR-.

In certain embodiments, the invention relates to any one of the aforementioned methods, wherein R is -H.

In certain embodiments, the invention relates to any one of the aforementioned methods, wherein X is chloro.

In certain embodiments, the invention relates to any one of the aforementioned methods, wherein Y¹ is -NH-.

In certain embodiments, the invention relates to any one of the aforementioned methods, wherein Y² is =N-.

In certain embodiments, the invention relates to any one of the aforementioned methods, wherein the agent is a compound of formula **IX**: 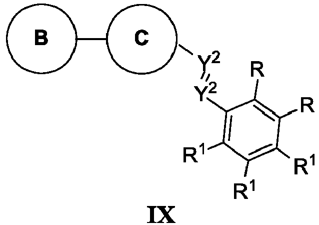 wherein, independently for each occurrence, 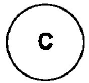
is a five-membered, unsaturated heterocycle diradical; 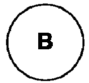
is heteroaryl;
R is -H, halo, aryl, nitro, alkyl, hydroxy, alkoxy, or amino;
R¹ is hydroxy, alkoxy, or amino; and
Y² is =N- or =CR-.

In certain embodiments, the invention relates to any one of the aforementioned methods, wherein 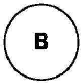 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned methods, wherein R is -H.

In certain embodiments, the invention relates to any one of the aforementioned methods, wherein R¹ is hydroxy.

In certain embodiments, the invention relates to any one of the aforementioned methods, wherein one instance of Y² is =N-.

In certain embodiments, the invention relates to any one of the aforementioned methods, wherein the agent is a compound of formula **X**: 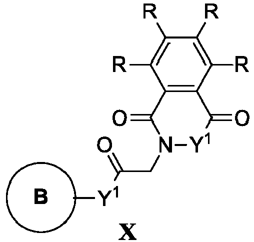 wherein, independently for each occurrence, 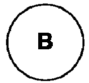
is heteroaryl;
R is -H, halo, aryl, nitro, alkyl, hydroxy, alkoxy, or amino;
Y¹ is -S-, -O-, or -NH-.

In certain embodiments, the invention relates to any one of the aforementioned methods, wherein 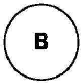 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned methods, wherein R is -H.

In certain embodiments, the invention relates to any one of the aforementioned methods, wherein Y¹ is -NH-.

In certain embodiments, the invention relates to any one of the aforementioned methods, wherein the agent is a compound of formula **XI**: 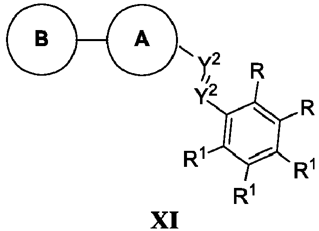 wherein, independently for each occurrence, 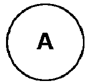
is an aryl heterocycle diradical; 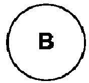
is heteroaryl;
R is -H, halo, aryl, nitro, alkyl, hydroxy, alkoxy, or amino;
R¹ is hydroxy, alkoxy, or amino; and
Y² is =N- or =CR-.

In certain embodiments, the invention relates to any one of the aforementioned methods, wherein 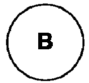 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.

In certain embodiments, the invention relates to any one of the aforementioned methods, wherein R is -H.

In certain embodiments, the invention relates to any one of the aforementioned methods, wherein R¹ is hydroxy.

In certain embodiments, the invention relates to any one of the aforementioned methods, wherein one instance of Y² is =N-.

In certain embodiments, the invention relates to any one of the aforementioned methods, wherein the agent is a compound of formula **XII**: 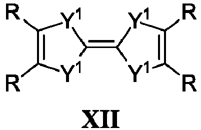 wherein, independently for each occurrence,
R is -H, halo, aryl, nitro, alkyl, hydroxy, alkoxy, or amino; and
Y¹ is -S-, -O-, or -NH-.

In certain embodiments, the invention relates to any one of the aforementioned methods, wherein at least one instance of R is -H.

In certain embodiments, the invention relates to any one of the aforementioned methods, wherein at least one instance of Y¹ is -S-.

In certain embodiments, the invention relates to any one of the aforementioned methods, wherein the agent is a compound of formula **XIII**: 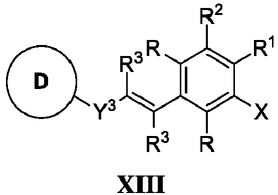 wherein, independently for each occurrence, 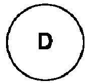
is aryl or heteroaryl;
R is -H, halo, aryl, nitro, alkyl, hydroxy, alkoxy, or amino;
R¹ is hydroxy, alkoxy, or amino;
R² is hydroxy, alkoxy, or amino;
R³ is -H, cyano, -CO₂R⁴, or -C(O)N(R⁴)₂;
X is halo;
Y³ is a bond, -C(O)-d, -C(O)NH-d, -NH-C(O)-d, or -C(O)NH-CH₂-d; and
d is a bond to

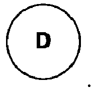

In certain embodiments, the invention relates to any one of the aforementioned methods, wherein R is -H

In certain embodiments, the invention relates to any one of the aforementioned methods, wherein R¹ is hydroxy.

In certain embodiments, the invention relates to any one of the aforementioned methods, wherein R² is alkoxy. In certain embodiments, the invention relates to any one of the aforementioned methods, wherein R² is methoxy.

In certain embodiments, the invention relates to any one of the aforementioned methods, wherein one instance of R³ is cyano.

In certain embodiments, the invention relates to any one of the aforementioned methods, wherein R³ is -H.

In certain embodiments, the invention relates to any one of the aforementioned methods, wherein X is bromo.

In certain embodiments, the invention relates to any one of the aforementioned methods, wherein Y³ is a bond. In certain embodiments, the invention relates to any one of the aforementioned methods, wherein Y³ is -C(O)-d. In certain embodiments, the invention relates to any one of the aforementioned methods, wherein Y³ is -C(O)NH-d. In certain embodiments, the invention relates to any one of the aforementioned methods, wherein Y³ is -NH-C(O)-d. In certain embodiments, the invention relates to any one of the aforementioned methods, wherein Y³ is -C(O)NH-CH₂-d.

In certain embodiments, the invention relates to any one of the aforementioned methods, wherein the agent is a compound of formula **XIV**: 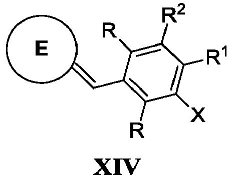 wherein, independently for each occurrence, 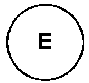
is a five-membered heterocycle radical;
R is -H, halo, aryl, nitro, alkyl, hydroxy, alkoxy, or amino;
R¹ is hydroxy, alkoxy, or amino;
R² is hydroxy, alkoxy, or amino; and
X is halo.

In certain embodiments, the invention relates to any one of the aforementioned methods, wherein R is -H

In certain embodiments, the invention relates to any one of the aforementioned methods, wherein R¹ is hydroxy.

In certain embodiments, the invention relates to any one of the aforementioned methods, wherein R² is alkoxy. In certain embodiments, the invention relates to any one of the aforementioned methods, wherein R² is methoxy.

In certain embodiments, the invention relates to any one of the aforementioned methods, wherein X is bromo.

In certain embodiments, the invention relates to any one of the aforementioned methods, wherein the agent is a compound of formula **XV**: 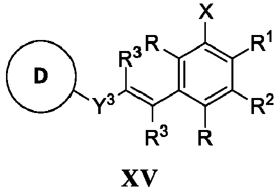 wherein, independently for each occurrence, 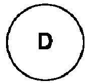
is aryl or heteroaryl;
R is -H, halo, aryl, nitro, alkyl, hydroxy, alkoxy, or amino;
R¹ is hydroxy, alkoxy, or amino;
R² is hydroxy, alkoxy, or amino;
R³ is -H, cyano, -CO₂R⁴, or -C(O)N(R⁴)₂;
X is halo;
Y³ is a bond, -C(O)-d, -C(O)NH-d, -NH-C(O)-d, or -C(O)NH-CH₂-d; and
d is a bond to

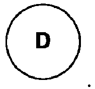

In certain embodiments, the invention relates to any one of the aforementioned methods, wherein R is -H

In certain embodiments, the invention relates to any one of the aforementioned methods, wherein R¹ is hydroxy.

In certain embodiments, the invention relates to any one of the aforementioned methods, wherein R² is alkoxy. In certain embodiments, the invention relates to any one of the aforementioned methods, wherein R² is methoxy.

In certain embodiments, the invention relates to any one of the aforementioned methods, wherein one instance of R³ is cyano.

In certain embodiments, the invention relates to any one of the aforementioned methods, wherein R³ is -H.

In certain embodiments, the invention relates to any one of the aforementioned methods, wherein X is bromo.

In certain embodiments, the invention relates to any one of the aforementioned methods, wherein Y³ is a bond. In certain embodiments, the invention relates to any one of the aforementioned methods, wherein Y³ is -C(O)-d. In certain embodiments, the invention relates to any one of the aforementioned methods, wherein Y³ is -C(O)NH-d. In certain embodiments, the invention relates to any one of the aforementioned methods, wherein Y³ is -NH-C(O)-d. In certain embodiments, the invention relates to any one of the aforementioned methods, wherein Y³ is -C(O)NH-CH₂-d.

In certain embodiments, the invention relates to any one of the aforementioned methods, wherein the agent is

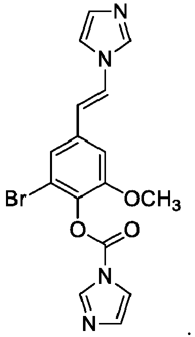

In certain embodiments, the invention relates to any one of the aforementioned methods, wherein the agent is selected from the group consisting of

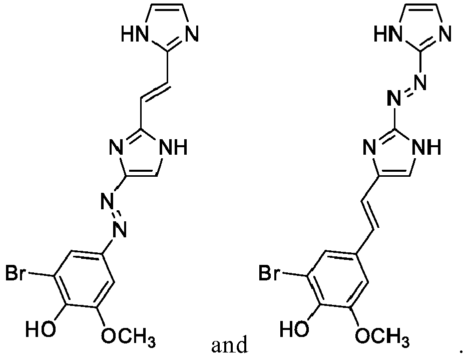

In certain embodiments, the invention relates to any one of the aforementioned methods, wherein the agent is selected from the group consisting of 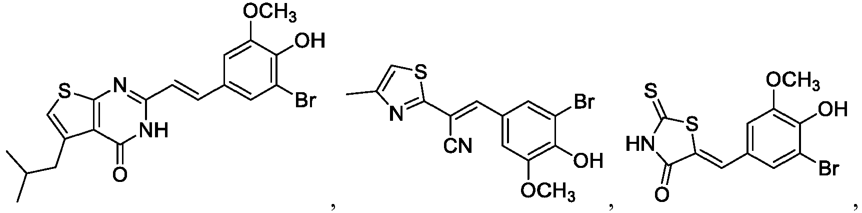 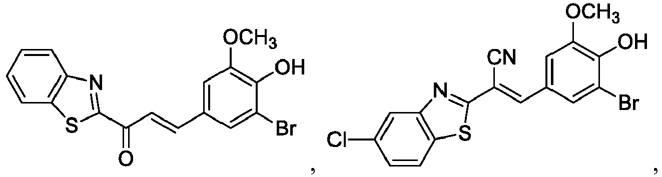 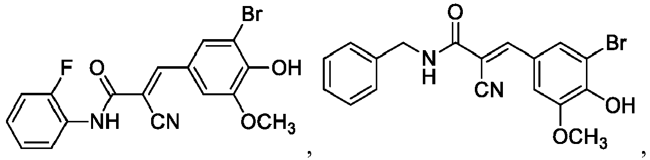 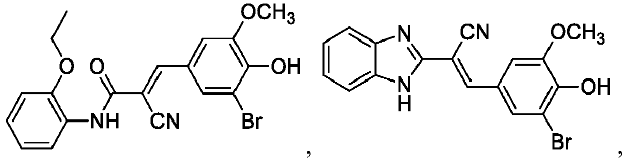 and

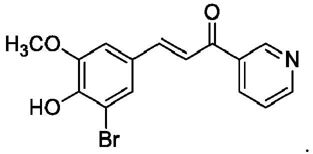

In certain embodiments, the invention relates to any one of the aforementioned methods, wherein the agent is selected from the group consisting of

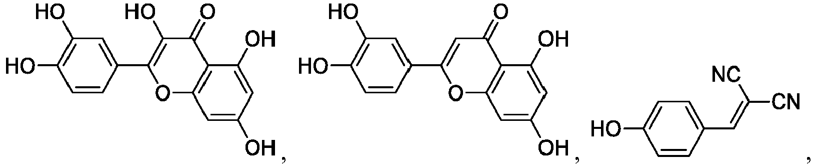

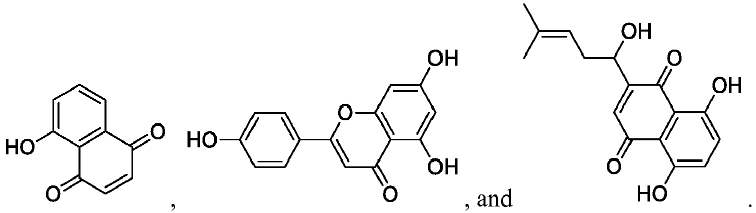

In certain embodiments, the invention relates to any one of the aforementioned methods, wherein the agent is selected from the group consisting of

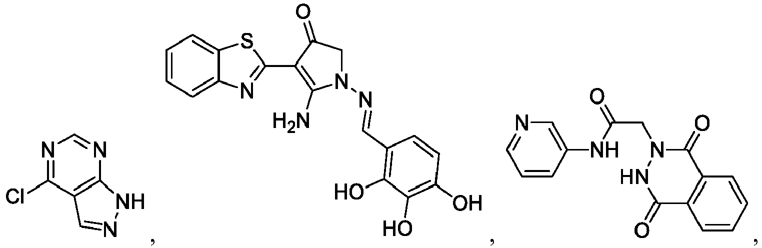

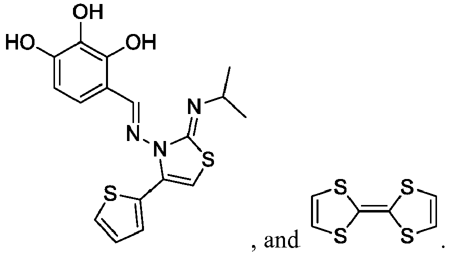

In certain embodiments, the invention relates to any one of the aforementioned methods, wherein the agent is selected from the group consisting of α-lineolic acid, lineolic acid, stearic acid, elaidic acid, arachidonic acid, oleic acid, and palmitoleic acid.

In certain embodiments, the invention relates to any one of the aforementioned methods, wherein the agent is selected from the group consisting of tryptophan, quinolinic acid, nicotinamide, nicotinamide mononucleotide, nicotinamide riboside, and nicotinic acid.

In certain embodiments, the invention relates to any one of the aforementioned methods, wherein the agent is selected from the group consisting of the compounds outlined in Table 1.

**Table 1**

| **Compound:** |
|---|
| Resveratrol (3,5,4'-Trihydroxy-*trans*-stilbene) |
| Butein (3,4,2',4'-Tetrahydroxychalcone) |
| Piceatannol (3,5,3',4'-Tetrahydroxy-*trans*stilbene) |
| Isoliquiritigen (4,2',4'-Trihydroxychalcone) |
| Fisetin (3,7,3',4'-Tetrahydroxyflavone) |
| 5,7,3',4',5'-Pentahydroxyflavone |
| Luteolin (5,7,3',4'-Tetrahydroxyflavone) |
| 3,6,3',4'-Tetrahydroxyflavone |
| Quercetin (3,5,7,3',4'-Pentahydroxyflavone) |
| 7,3',4',5'-Tetrahydroxyflavone |
| Kaempferol (3,5,7,4'-Tetrahydroxyflavone) |
| 6-Hydroxyapigenin (5,6,7,4'-Tetrahydroxyflavone; Scutellarein) |
| 3,4,2',4',6'-Pentahydroxychalcone |
| Apigenin (5,7,4'-Trihydroxyflavone) |
| Hinokitiol (b-Thujaplicin; 2-hydroxy-4-isopropyl-2,4,6-cycloheptatrien-1-one) |
| Daidzein (7,4'-Dihydroxyisoflavone) |
| Naringenin (5,7,4'-Trihydroxyflavanone) |
| 3,6,2',4'-Tetrahydroxyflavone |
| L-(+)-Ergothioneine ((S)-a-Carboxy-2,3-dihydro-N,N,N-trimethyl-2-thioxo-1Himidazole-4-ethanaminium inner salt) |
| 3,5,7,3',4'-Pentahydroxyflavanone |
| Deoxyrhapontin (3,5-Dihydroxy-4'-methoxystilbene 3-O-β-D-glucoside) |
| Flavanone |
| 7,8,3',4'-Tetrahydroxyflavone |
| 7,4'-Dihydroxyflavone |
| Caffeic Acid Phenyl Ester |
| 3,6,2',3'-Tetrahydroxyflavone |
| 4'-Hydroxyflavone |
| Pelargonidin chloride (3,5,7.4'-Tetrahydroxyflavylium chloride) |
| 5,4'-Dihydroxyflavone |
| (-)-Epicatechin (Hydroxy Sites: 3,5,7,3',4') |
| 5,7-Dihydroxyflavone |
| *trans*-Stilbene |
| Morin (3,5,7,2',4'-Pentahydroxyflavone) |
| Flavone |
| (-)-Catechin (Hydroxy Sites: 3,5,7,3',4') |
| Rhapontin (3,3',5-Trihydroxy-4'-methoxystilbene 3-O-B-β-glucoside) |
| (-)-Gallocatechin (Hydroxy Sites: 3,5,7,3',4',5') |
| Chalcone |
| (+)-Catechin (Hydroxy Sites: 3,5,7,3',4') |
| (+)-Epicatechin (Hydroxy Sites: 3,5,7,3',4') |
| MCI-186 (3-Methyl-1-phenyl-2-pyrazolin-5-one) |
| 5-Hydroxyflavone |
| HBED (N,N'-Di-(2-hydroxybenzyl)ethylenediamine-N,N'-diacetic acid•HCl•H2O) |
| *cis*-Stilbene |
| Genistein (5,7,4'-Trihydroxyisoflavone) |
| Ambroxol (trans-4-(2-Amino-3,5-dibromobenzylamino) cyclohexane•HCl) |
| U-83836E ((-)-2-((4-(2,6-di-1-Pyrrolidinyl-4-pyrimidinyl)-1-piperazinyl)methyl)-3,4-dihydro-2,5,7,8-tetramethyl-2H-1-benzopyran-6-ol•2HCl) |

In certain embodiments, the invention relates to any one of the aforementioned methods, wherein the agent is a CD38 inhibitor.

In certain embodiments, the invention relates to any one of the aforementioned methods, wherein the CD 38 inhibitor is selected from the group consisting of the compounds outlined in Table 2.

**Table 2: CD38 Inhibitors**

| |
|---|
| 1-[(2-Acetoxyethoxy)methyl]-3-(aminocarbonyl)-pyridinium chloride |
| 1-[(2-Benzyloxyethoxy)methyl]-3-(aminocarbonyl)-pyridinium chloride |
| 1-{[2-(4-Methoxy-phenoxy)ethoxy]methyl}-3-(aminocarbonyl)-pyridinium chloride |
| 1-{[2-(4-Phenoxy-phenoxy)ethoxy]methyl}-3-(aminocarbonyl)-pyridinium chloride |
| 1-{[2-(4-Nitro-phenoxy)ethoxy]methyl}-3-(aminocarbonyl)-pyridinium chloride |
| 1-{[2-(3-Trifluoromethyl-phenoxy)ethoxy]methyl}-3-(aminocarbonyl)-pyridiniumchloride |
| 1-{[2-(8'-Quinolyloxy)ethoxy]methyl}-3-(aminocarbonyl)-pyridinium chloride |
| 1,2-Dimethoxy-ethylene-bis-N,N'-3-(aminocarbonyl)-pyridinium dichloride |
| 1,4-Dimethoxy-butylene-bis-N,N'-3-(aminocarbonyl)-pyridinium dichloride |
| 1,4-Dimethoxy-butyne-bis-N,N'-3-(aminocarbonyl)-pyridinium dichloride |
| 1,4-Dimethoxy-hexamethylene-bis-N,N'-3-(aminocarbonyl)- pyridinium dichloride |
| (*E*)-1-{[4-(8'-Quinolyloxy)but-2-enyloxy]methyl}-3-(aminocarbonyl)-pyridinium chloride |
| 1-{[2-(4-Phenoxy-phenoxy)ethoxy]methyl}-6-(aminocarbonyl)-quinolinium chloride |
| 1-{[2-(4-Phenoxy-phenoxy)ethoxy]methyl}-3-(aminocarbonyl)-4-amino-pyridinium chloride |
| Luteolinidin |
| Kuromanin |
| Luteolin |
| Delphinidin |
| Pelargonidin |
| Malvidin |
| Quercetagetinidin |
| Peonidin |
| Myricetin |
| Cyanidin |
| Diosmetinidin |
| Quercetin |
| Robinetin |
| Petunidin |
| Fisetinidin |
| Quercetagetin |
| *rac*-Taxifolin |
| *rac*-Catechin |
| Piceatannol |
| *trans*-Resveratrol |

In certain embodiments, the invention relates to any one of the aforementioned methods, wherein the agent is a substrate for the citric acid cycle.

In certain embodiments, the invention relates to any one of the aforementioned methods, wherein the agent is selected from the group consisting of pyruvate, oxaloacetate, acetyl CoA, citrate, cis-aconitate, isocitrate, oxalosuccinate, alpha-ketoglutarate, succinyl-CoA, guanosine diphosphate, succinate, ubiquinone, fumarate, and L-malate.

In certain embodiments, the invention relates to a method of increasing NAD+ levels in a cell, comprising the step of
contacting the cell with a precursor of NAD+.

In certain embodiments, the invention relates to any one of the aforementioned methods, wherein the precursor of NAD+ is nicotinamide mononucleotide. See Figure 7.

In certain embodiments, the invention relates to any one of the aforementioned methods, wherein the precursor of NAD+ is nicotinamide.

In certain embodiments, the invention relates to a method of increasing NAD+ levels in a cell, comprising the step of
delivering to the cell in vivo a precursor of NAD+.

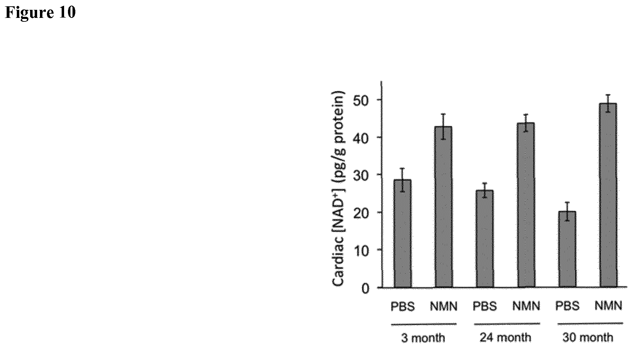

In certain embodiments, the invention relates to any one of the aforementioned methods, wherein the precursor of NAD+ is nicotinamide mononucleotide. See Figure 10.

In certain embodiments, the invention relates to any one of the aforementioned methods, wherein the precursor of NAD+ is nicotinamide.

In certain embodiments, the invention relates to a method of increasing NAD+ levels in a cell, comprising the step of
increasing the expression of a gene, wherein the gene synthesizes NAD+.

In certain embodiments, the invention relates to any one of the aforementioned methods, wherein the gene is NAMPT or NMNAT1-3.

In certain embodiments, the invention relates to a method of increasing NAD+ levels in a cell, comprising the step of
inhibiting the degradation of NAD+.

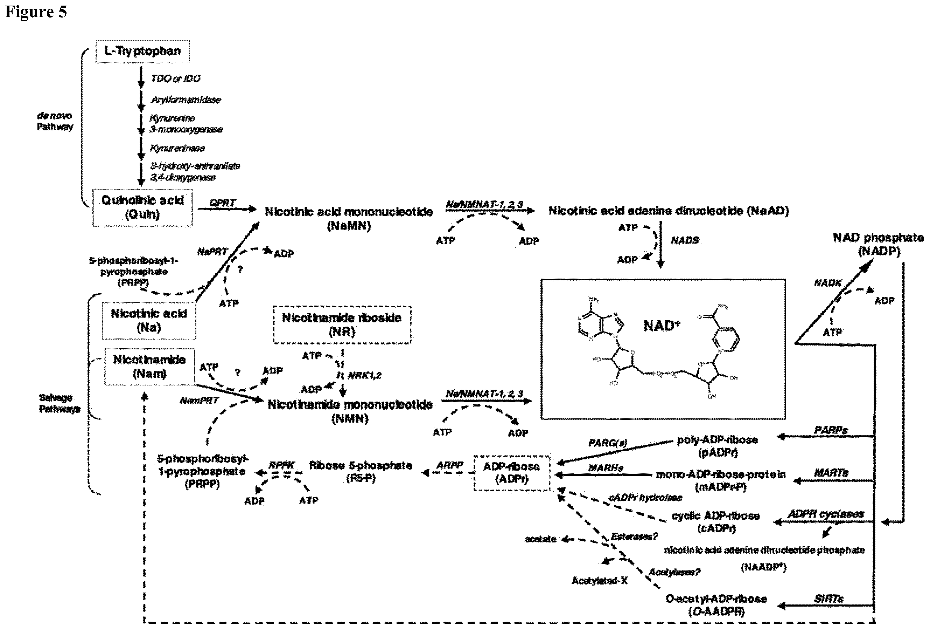

In certain embodiments, the invention relates to any one of the aforementioned methods, wherein a PARP is inhibited. See Figure 5.

In certain embodiments, the invention relates to any one of the aforementioned methods, wherein CD38 is inhibited. See Figure 5.

In certain embodiments, the invention relates to any one of the aforementioned methods, wherein the rate of the citric acid cycle of the cell is inhibited.

In certain embodiments, the invention relates to any one of the aforementioned methods, wherein the rate of the citric acid cycle of the cell is inhibited by the addition of an agent selected from the group consisting of pyruvate, oxaloacetate, acetyl CoA, citrate, cis-aconitate, isocitrate, oxalosuccinate, alpha-ketoglutarate, succinyl-CoA, guanosine diphosphate, succinate, ubiquinone, fumarate, and L-malate.

Pharmaceutically acceptable salts and prodrugs of the compounds described herein may also be used.

In another embodiment, cells obtained from a subject, e.g., a human or other mammal, are treated according to the methods of the invention and then administered to the same or a different subject. Accordingly, cells or tissues obtained from a donor for use as a graft can be treated as described herein prior to administering to the recipient of the graft. For example, bone marrow cells can be obtained from a subject, treated *ex vivo* and then administered to a recipient.

In yet other embodiments, cells are treated *in vivo.* For example, skin can be protected from aging, e.g., developing wrinkles, by treating skin, e.g., epithelial cells, as described herein.

Compounds can also be delivered to a tissue or organ within a subject, such as by injection.

In yet another embodiment, an agent of the invention is administered to subjects, such as to generally increase the life span of its cells, protect its cells against certain types of stresses, to prevent or treat diseases of aging, the process of aging itself, diseases or afflictions associate with cell death, infection and toxic agents. For example, an agent can be taken by subjects as food supplements. In one embodiment, such an agent is a component of a multi-vitamin complex.

All animals typically go through a period of growth and maturation followed by a period of progressive and irreversible physiological decline ending in death. The length of time from birth to death is known as the life span of an organism, and each organism has a characteristic average life span. Aging is a physical manifestation of the changes underlying the passage of time as measured by percent of average life span.

In some cases, characteristics of aging can be quite obvious. For example, characteristics of older humans include skin wrinkling, graying of the hair, baldness, and cataracts, as well as hypermelanosis, osteoporosis, cerebral cortical atrophy, lymphoid depletion, thymic atrophy, erectile dysfunction, increased incidence of diabetes type II, atherosclerosis, cancer, and heart disease. Other aspects of mammalian aging include weight loss, lordokyphosis (hunchback spine), absence of vigor, lymphoid atrophy, decreased bone density, dermal thickening and subcutaneous adipose tissue, decreased ability to tolerate stress (including heat or cold, wounding, anesthesia, and hematopoietic precursor cell ablation), liver pathology, atrophy of intestinal villi, skin ulceration, amyloid deposits, and joint diseases.

Careful observation reveals characteristics of aging in other eukaryotes, including invertebrates. For example, characteristics of aging in the model organism *C. elegans* include slow movement, flaccidity, yolk accumulation, intestinal autofluorescence (lipofuscin), loss of ability to eat food or dispel waste, necrotic cavities in tissues, and germ cell appearance.

Those skilled in the art will recognize that the aging process is also manifested at the cellular level, as well as in mitochondria. A loss of mitochondrial NAD+ is known to increase cell death and age-related diseases, such as cardiac hypertrophy. Cellular aging is manifested in loss of doubling capacity, increased levels of apoptosis, changes in differentiated phenotype, and changes in metabolism, e.g., decreased levels of protein synthesis and turnover.

Given the programmed nature of cellular and organismal aging, it is possible to evaluate the "biological age" of a cell or organism by means of phenotypic characteristics that are correlated with aging. For example, biological age can be deduced from patterns of gene expression, resistance to stress (e.g., oxidative or genotoxic stress), rate of cellular proliferation, and the metabolic characteristics of cells (e.g., rates of protein synthesis and turnover, mitochondrial function, ubiquinone biosynthesis, cholesterol biosynthesis, ATP levels within the cell, levels of a Krebs cycle intermediate in the cell, glucose metabolism, nucleic acid metabolism, ribosomal translation rates, etc.). As used herein, "biological age" is a measure of the age of a cell or organism based upon the molecular characteristics of the cell or organism. Biological age is distinct from "temporal age," which refers to the age of a cell or organism as measured by days, months, and years.

The rate of aging of an organism, e.g., an invertebrate (e.g., a worm or a fly) or a vertebrate (e.g., a rodent, e.g., a mouse) can be determined by a variety of methods, e.g., by one or more of: a) assessing the life span of the cell or the organism; (b) assessing the presence or abundance of a gene transcript or gene product in the cell or organism that has a biological age-dependent expression pattern; (c) evaluating resistance of the cell or organism to stress, e.g., genotoxic stress (e.g., etopicide, UV irradiation, exposure to a mutagen, and so forth) or oxidative stress; (d) evaluating one or more metabolic parameters of the cell or organism; (e) evaluating the proliferative capacity of the cell or a set of cells present in the organism; and (f) evaluating physical appearance or behavior of the cell or organism. In one example, evaluating the rate of aging includes directly measuring the average life span of a group of animals (e.g., a group of genetically matched animals) and comparing the resulting average to the average life span of a control group of animals (e.g., a group of animals that did not receive the test compound but are genetically matched to the group of animals that did receive the test compound). Alternatively, the rate of aging of an organism can be determined by measuring an age-related parameter. Examples of age-related parameters include: appearance, e.g., visible signs of age; the expression of one or more genes or proteins (e.g., genes or proteins that have an age-related expression pattern); resistance to oxidative stress; metabolic parameters (e.g., protein synthesis or degradation, ubiquinone biosynthesis, cholesterol biosynthesis, ATP levels, glucose metabolism, nucleic acid metabolism, ribosomal translation rates, etc.); and cellular proliferation (e.g., of retinal cells, bone cells, white blood cells, etc.).

Agents that extend the life span of cells and protect them from stress can also be administered to subjects for treatment of diseases, e.g., chronic diseases, associated with cell death, such as to protect the cells from cell death, e.g., diseases associated with neural cell death or muscular cell death. In particular, based at least on the fact that the proteins involve protect neurons from axonal degeneration, the methods may be used to prevent or alleviate neurodegeneration and peripheral neuropathies associated with chemotherapy, such as cancer chemotherapy (e.g., taxol or cisplatin treatment). Neurodegenerative diseases include Parkinson's disease, Alzheimer's disease, multiple sclerosis, amniotropic lateral sclerosis (ALS), Huntington's disease and muscular dystrophy. Thus, the agents may be used as neuroprotective agents. The agent may be administered in the tissue or organ likely to encounter cell death.

Such agents can also be administered to a subject suffering from an acute damage to an organ or tissue, e.g., a subject suffering from stroke or myocardial infarction or a subject suffering from a spinal cord injury. Agents can also be used to repair an alcoholic's liver.

More generally, agents described herein may be administered to subjects in which caloric restriction or the effects thereof would be beneficial. Subjects may be subjects suffering from an aging disease, e.g., stroke, heart disease, arthritis, high blood pressure. They may also be administered for treating a metabolic disease, such as insulin-resistance or other precursor symptom of type II diabetes, type II diabetes or complications thereof. Methods may increase insulin sensitivity or decrease insulin levels in a subject. A method may comprise administering to a subject, such as a subject in need thereof, a pharmaceutically effective amount of an agent that increases the activity or protein level of a protein involved in the NAD+ salvage pathway, i.e., in the synthesis of NAD+ and the degradation of nicotinamide. A subject in need of such a treatment may be a subject who has insulin resistance or other precursor symptom of type II diabetes, who has type II diabetes, or who is likely to develop any of these conditions. For example, the subject may be a subject having insulin resistance, e.g., having high circulating levels of insulin and/or associated conditions, such as hyperlipidemia, dyslipogenesis, hypercholesterolemia, impaired glucose tolerance, high blood glucose sugar level, other manifestations of syndrome X, hypertension, atherosclerosis and lipodystrophy.

Yet other disorders that may be treated with agents of the invention include restenosis, e.g., following coronary intervention, and disorders relating to an abnormal level of high density and low density cholesterol.

Based at least on the fact that the enzymes involved deacetylate and regulate NF-kB, the methods described herein may be used to treat inflammatory conditions, such as arthritis, Crohn's disease, rheumatoid arthritis, asthma, atherosclerosis, coronary heart disease, reperfusion injury from heart attack or stroke, ulcerative colitis, and active inflammatory bowel disease (IBD).

Other conditions that can be treated include ocular disorders, e.g., associated with the aging of the eye, such as dry eye, cataracts, glaucoma, and macular degeneration. They can also be used for treatment of diseases, e.g., AIDS; fulminant hepatitis; diseases linked to degeneration of the brain, such as Creutzfeld-Jakob disease, retinitis pigmentosa and cerebellar degeneration; myelodysplasis such as aplastic anemia; ischemic diseases such as myocardial infarction and stroke; hepatic diseases such as alcoholic hepatitis, hepatitis B and hepatitis C; joint-diseases such as osteoarthritis; atherosclerosis; alopecia; damage to the skin due to UV light; lichen planus; atrophy of the skin; cataract; and graft rejections.

Based at least on the fact that enzymes involved have been shown to be involved in fat mobilization, e.g., by repressing PPAR-γ, methods described herein for mimicking calorie restriction can also be used for stimulating fat mobilization, e.g., for treating obesity and any condition resulting therefrom or for reducing weight gain.

In addition, the agents described herein may be administered to subjects for protection against or treatment of exposure to toxic agents, radiation or any warfare chemical. For example, the agents may be administered to subjects who have recently received or are likely to receive a dose of radiation. In one embodiment, the dose of radiation is received as part of a work-related or medical procedure, e.g., working in a nuclear power plant, flying an airplane, an X-ray, CAT scan, or the administration of a radioactive dye for medical imaging; in such an embodiment, the agent is administered as a prophylactic measure. In another embodiment, the radiation exposure is received unintentionally, e.g., as a result of an industrial accident, terrorist act, or act of war involving radioactive material. In such a case, the agent would be administered as soon as possible after the exposure to inhibit apoptosis and the subsequent development of acute radiation syndrome. The agents described herein could also be used to protect non-cancerous cells from the effects of chemotherapy, such as to protect neurons in the case of preventing neuropathies, hematoxicity, renal toxicity, and gastrointestinal toxicity due to chemotherapy.

In certain embodiments, the invention relates to a method of promoting DNA repair in cells. Accordingly, cells exposed to conditions that may trigger DNA damage, e.g., U.S. radiation and ethidium bromide, may be protected by contacting them before, during and/or after exposure to the DNA damaging agent, with an agent of the invention.

In other embodiments, the methods of the invention are applied to yeast cells. Situations in which it may be desirable to extend the life span of yeast cells and to protect them against certain types of stress include any process in which yeast is used, e.g., the making of beer, yogurt, and bakery, e.g., making of bread. Use of yeast having an extended life span can result in using less yeast or in having the yeast be active for longer periods of time.

The agents described herein may also be used to mimic calorie restriction in plants, e.g., to increase lifespan, stress resistance, and resistance to apoptosis in plants. In one embodiment, an agent is applied to plants, either on a periodic basis or in times of stress, e.g., drought, frost, or an infestation of insects or fungi. In another embodiment, plants are genetically modified to produce an agent. In another embodiment, plants and fruits are treated with an agent prior to picking and shipping to increase resistance to damage during shipping.

The agents may also be used to increase lifespan, stress resistance and resistance to apoptosis in insects. In this embodiment, the agents would be applied to useful insects, e.g., bees and other insects that are involved in pollination of plants. In a specific embodiment, an agent would be applied to bees involved in the production of honey.

Subjects that may be treated as described herein include eukaryotes, such as mammals, e.g., humans, ovines, bovines, equines, porcines, canines, felines, non-human primate, mice, and rats. Cells that may be treated include eukaryotic cells, e.g., from a subject described above, or plant cells, yeast cells and prokaryotic cells, e.g., bacterial cells.

### Pharmaceutical compositions and methods

Pharmaceutical agents for use in accordance with the present methods may be formulated in conventional manner using one or more physiologically acceptable carriers or excipients. Thus, compounds or agents that increase the protein or expression level of nucleic acids described herein, and their physiologically acceptable salts and solvates may be formulated for administration by, for example, injection, inhalation or insufflation (either through the mouth or the nose) or oral, buccal, parenteral or rectal administration. In one embodiment, the agent is administered locally, e.g., at the site where the target cells are present, such as by the use of a patch.

Agents can be formulated for a variety of loads of administration, including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous. For injection, the agents can be formulated in liquid solutions, preferably in physiologically compatible buffers such as Hank's solution or Ringer's solution. In addition, the agents may be formulated in solid form and redissolved or suspended immediately prior to use. Lyophilized forms are also included.

For oral administration, the pharmaceutical compositions may take the form of, for example, tablets, lozanges, or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., ationd oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate. Preparations for oral administration may be suitably formulated to give controlled release of the active compound.

Agents that may oxidize and lose biological activity, especially in a liquid or semisolid form, may be prepared in a nitrogen atmosphere or sealed in a type of capsule and/or foil package that excludes oxygen (e.g. Capsugel™).

For administration by inhalation, the agents may be conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin, for use in an inhaler or insufflator may be formulated containing a powder mix of the agent and a suitable powder base such as lactose or starch.

The agents may be formulated for parenteral administration by injection, e.g., by bolus injection or continuous infusion. Formulations for injection may be presented in unit dosage form, e.g., in ampoules or in multi-dose containers, with an added preservative. The agents may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The agents may also be formulated in rectal compositions such as suppositories or retention enemas, e.g., containing conventional suppository bases such as cocoa butter or other glycerides.

In addition to the formulations described previously, the agents may also be formulated as a depot preparation. Such long acting formulations may be administered by implantation (for example subcutaneously or intramuscularly) or by intramuscular injection. Thus, for example, the agents may be formulated with suitable polymeric or hydrophobic materials (for example as an emulsion in an acceptable oil) or ion exchange resins, or as sparingly soluble derivatives, for example, as a sparingly soluble salt. Controlled release formulas also include patches, e.g., transdermal patches. Patches may be used with a sonic applicator that deploys ultrasound in a unique combination of waveforms to introduce drug molecules through the skin that normally could not be effectively delivered transdermally.

Pharmaceutical compositions may comprise from about 0.00001 to 100% such as from 0.001 to 10% or from 0.1% to 5% by weight of one or more agents described herein.

In one embodiment, an agent described herein, is incorporated into a topical formulation containing a topical carrier that is generally suited to topical drug administration and comprising any such material known in the art. The topical carrier may be selected so as to provide the composition in the desired form, e.g., as an ointment, lotion, cream, microemulsion, gel, oil, solution, or the like, and may be comprised of a material of either naturally occurring or synthetic origin. It is preferable that the selected carrier not adversely affect the active agent or other components of the topical formulation. Examples of suitable topical carriers for use herein include water, alcohols and other nontoxic organic solvents, glycerin, mineral oil, silicone, petroleum jelly, lanolin, fatty acids, vegetable oils, parabens, waxes, and the like.

Formulations may be colorless, odorless ointments, lotions, creams, microemulsions and gels.

Agents may be incorporated into ointments, which generally are semisolid preparations which are typically based on petrolatum or other petroleum derivatives. The specific ointment base to be used, as will be appreciated by those skilled in the art, is one that will provide for optimum drug delivery, and, preferably, will provide for other desired characteristics as well, e.g., emolliency or the like. As with other carriers or vehicles, an ointment base should be inert, stable, nonirritating and nonsensitizing. As explained in Remington's, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin and stearic acid. Exemplary water-soluble ointment bases are prepared from polyethylene glycols (PEGs) of varying molecular weight; again, reference may be had to Remington's, *supra*, for further information.

Agents may be incorporated into lotions, which generally are preparations to be applied to the skin surface without friction, and are typically liquid or semiliquid preparations in which solid particles, including the active agent, are present in a water or alcohol base. Lotions are usually suspensions of solids, and may comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethylcellulose, or the like. An exemplary lotion formulation for use in conjunction with the present method contains propylene glycol mixed with a hydrophilic petrolatum such as that which may be obtained under the trademark Aquaphor™ from Beiersdorf, Inc. (Norwalk, Conn.).

Agents may be incorporated into creams, which generally are viscous liquid or semisolid emulsions, either oil-in-water or water-in-oil. Cream bases are water-washable, and contain an oil phase, an emulsifier and an aqueous phase. The oil phase is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation, as explained in Remington's, *supra*, is generally a nonionic, anionic, cationic or amphoteric surfactant.

Agents may be incorporated into microemulsions, which generally are thermodynamically stable, isotropically clear dispersions of two immiscible liquids, such as oil and water, stabilized by an interfacial film of surfactant molecules (Encyclopedia of Pharmaceutical Technology (New York: Marcel Dekker, 1992), volume 9). For the preparation of microemulsions, surfactant (emulsifier), co-surfactant (co-emulsifier), an oil phase and a water phase are necessary. Suitable surfactants include any surfactants that are useful in the preparation of emulsions, e.g., emulsifiers that are typically used in the preparation of creams. The co-surfactant (or "co-emulsifer") is generally selected from the group of polyglycerol derivatives, glycerol derivatives and fatty alcohols. Preferred emulsifier/co-emulsifier combinations are generally although not necessarily selected from the group consisting of: glyceryl monostearate and polyoxyethylene stearate; polyethylene glycol and ethylene glycol palmitostearate; and caprilic and capric triglycerides and oleoyl macrogolglycerides. The water phase includes not only water but also, typically, buffers, glucose, propylene glycol, polyethylene glycols, preferably lower molecular weight polyethylene glycols (e.g., PEG 300 and PEG 400), and/or glycerol, and the like, while the oil phase will generally comprise, for example, fatty acid esters, modified vegetable oils, silicone oils, mixtures of mono- di- and triglycerides, mono- and di-esters of PEG (e.g., oleoyl macrogol glycerides), etc.

Agents may be incorporated into gel formulations, which generally are semisolid systems consisting of either suspensions made up of small inorganic particles (two-phase systems) or large organic molecules distributed substantially uniformly throughout a carrier liquid (single phase gels). Single phase gels can be made, for example, by combining the active agent, a carrier liquid and a suitable gelling agent such as tragacanth (at 2 to 5%), sodium alginate (at 2-10%), gelatin (at 2-15%), methylcellulose (at 3-5%), sodium carboxymethylcellulose (at 2-5%), carbomer (at 0.3-5%) or polyvinyl alcohol (at 10-20%) together and mixing until a characteristic semisolid product is produced. Other suitable gelling agents include methylhydroxycellulose, polyoxyethylene-polyoxypropylene, hydroxyethylcellulose and gelatin. Although gels commonly employ aqueous carrier liquid, alcohols and oils can be used as the carrier liquid as well.

Various additives, known to those skilled in the art, may be included in formulations, e.g., topical formulations. Examples of additives include, but are not limited to, solubilizers, skin permeation enhancers, opacifiers, preservatives (e.g., anti-oxidants), gelling agents, buffering agents, surfactants (particularly nonionic and amphoteric surfactants), emulsifiers, emollients, thickening agents, stabilizers, humectants, colorants, fragrance, and the like. Inclusion of solubilizers and/or skin permeation enhancers is particularly preferred, along with emulsifiers, emollients and preservatives. An optimum topical formulation comprises approximately: 2 wt. % to 60 wt. %, preferably 2 wt. % to 50 wt. %, solubilizer and/or skin permeation enhancer; 2 wt. % to 50 wt. %, preferably 2 wt. % to 20 wt. %, emulsifiers; 2 wt. % to 20 wt. % emollient; and 0.01 to 0.2 wt. % preservative, with the active agent and carrier (e.g., water) making of the remainder of the formulation.

A skin permeation enhancer serves to facilitate passage of therapeutic levels of active agent to pass through a reasonably sized area of unbroken skin. Suitable enhancers are well known in the art and include, for example: lower alkanols such as methanol ethanol and 2-propanol; alkyl methyl sulfoxides such as dimethylsulfoxide (DMSO), decylmethylsulfoxide (C¹⁰ MSO) and tetradecylmethyl sulfoxide; pyrrolidones such as 2-pyrrolidone, N-methyl-2-pyrrolidone and N-(-hydroxyethyl)pyrrolidone; urea; N,N-diethyl-m-toluamide; C₂ -C₆ alkanediols; miscellaneous solvents such as dimethyl formamide (DMF), N,N-dimethylacetamide (DMA) and tetrahydrofurfuryl alcohol; and the 1-substituted azacycloheptan-2-ones, particularly 1-n-dodecylcyclazacycloheptan-2-one (laurocapram; available under the trademark AzoneRTM from Whitby Research Incorporated, Richmond, Va.).

Examples of solubilizers include, but are not limited to, the following: hydrophilic ethers such as diethylene glycol monoethyl ether (ethoxydiglycol, available commercially as Transcutol™) and diethylene glycol monoethyl ether oleate (available commercially as Softcutol™); polyethylene castor oil derivatives such as polyoxy 35 castor oil, polyoxy 40 hydrogenated castor oil, etc.; polyethylene glycol, particularly lower molecular weight polyethylene glycols such as PEG 300 and PEG 400, and polyethylene glycol derivatives such as PEG-8 caprylic/capric glycerides (available commercially as Labrasol™); alkyl methyl sulfoxides such as DMSO; pyrrolidones such as 2-pyrrolidone and N-methyl-2-pyrrolidone; and DMA. Many solubilizers can also act as absorption enhancers. A single solubilizer may be incorporated into the formulation, or a mixture of solubilizers may be incorporated therein.

Suitable emulsifiers and co-emulsifiers include, without limitation, those emulsifiers and co-emulsifiers described with respect to microemulsion formulations. Emollients include, for example, propylene glycol, glycerol, isopropyl myristate, polypropylene glycol-2 (PPG-2) myristyl ether propionate, and the like.

Other active agents may also be included in formulations, e.g., anti-inflammatory agents, analgesics, antimicrobial agents, antifungal agents, antibiotics, vitamins, antioxidants, and sunblock agents commonly found in sunscreen formulations including, but not limited to, anthranilates, benzophenones (particularly benzophenone-3), camphor derivatives, cinnamates (e.g., octyl methoxycinnamate), dibenzoyl methanes (e.g., butyl methoxydibenzoyl methane), p-aminobenzoic acid (PABA) and derivatives thereof, and salicylates (e.g., octyl salicylate).

In certain topical formulations, the active agent is present in an amount in the range of approximately 0.25 wt. % to 75 wt. % of the formulation, preferably in the range of approximately 0.25 wt. % to 30 wt. % of the formulation, more preferably in the range of approximately 0.5 wt. % to 15 wt. % of the formulation, and most preferably in the range of approximately 1.0 wt. % to 10 wt. % of the formulation.

Topical skin treatment compositions can be packaged in a suitable container to suit its viscosity and intended use by the consumer. For example, a lotion or cream can be packaged in a bottle or a roll-ball applicator, or a propellant-driven aerosol device or a container fitted with a pump suitable for finger operation. When the composition is a cream, it can simply be stored in a non-deformable bottle or squeeze container, such as a tube or a lidded jar. The composition may also be included in capsules such as those described in U.S. Pat. No. 5,063,507. Accordingly, also provided are closed containers containing a cosmetically acceptable composition.

In an alternative embodiment, a pharmaceutical formulation is provided for oral or parenteral administration, in which case the formulation may comprise an activating compound-containing microemulsion as described above, and may contain alternative pharmaceutically acceptable carriers, vehicles, additives, etc. particularly suited to oral or parenteral drug administration. Alternatively, an activating compound-containing microemulsion may be administered orally or parenterally substantially as described above, without modification.

Administration of an agent may be followed by measuring a factor in the subject, such as measuring the level of NAD+, NAM, or ADPR. In an illustrative embodiment, a cell is obtained from a subject following administration of an agent to the subject, such as by obtaining a biopsy, and the factor is determined in the biopsy. Alternatively, biomarkers, such as plasma biomarkers may be followed. The cell may be any cell of the subject, but in cases in which an agent is administered locally, the cell is preferably a cell that is located in the vicinity of the site of administration.

Other factors that may be monitored include a symptom of aging, weight, body mass, blood glucose sugar levels, blood lipid levels and any other factor that may be measured for monitoring diseases or conditions described herein.

### Kits

Also provided herein are kits, e.g., kits for therapeutic purposes, including kits for modulating aging, apoptosis, and for treating diseases, e.g., those described herein. A kit may comprise one or more agent described herein, and optionally devices for contacting cells with the agents. Devices include syringes, stents and other devices for introducing an agent into a subject or applying it to the skin of a subject.

Further, a kit may also contain components for measuring a factor, e.g., described above, such as a protein or transcript level, e.g., in tissue samples.

### EXAMPLES

The present invention is further illustrated by the following examples which should not be construed as limiting in any way. The contents of all cited references (including literature references, issued patents, published patent applications and GenBank Accession numbers as cited throughout this application) are hereby expressly incorporated by reference.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization(B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### Example 1 - CD38 Inhibition

CD38 inhibition of commercial compounds was measured and is depicted in Figure 1 and Figure 2 and Figure 3A.

### Example 2 - Increase of NAD+ Levels

### Materials and Methods

Oogonial stem cells were isolated from dissociated ovaries using a FACS based sorting protocol to purify OSCs free of contaminating oocytes (White et al., in press). Cells were maintained in culture medium consisted of minimum essential medium α (MEMα), 10% FBS, 1 mM sodium pyruvate, 1 mM non-essential amino acids, 2 mM 1-glutamine, 0.1 mM β-mercaptoethanol (Sigma), 10 ng/ml-1 LIF (Millipore), 1X N-2 MAX Media Supplement (R&D) 10 ng/ml EGF (Epidermal growth factor, Recombinant human; Gibco), 40 ng/ml human GDNF (glial cell line-derived neurotrophic factor; R&D systems), 1 ng/ml human bFGF (basic fibroblast growth factor; Gibco)

For all experiments 25,000 cells were plated in each well of a 24 well plate. Cells were allowed to attach for 24h and then were treated with NMN (β-Nicotinamide mononucleotide; Sigma). Unless otherwise stated, NMN was added at for 12 and again for 6 hours before analysis (12 + 6 h).

### Mitochondrial DNA Copy number

Total cellular DNA was isolated from cells at the indicated time points using DNeasy Blood & Tissue Kit (Qiagen) according to the manufacturer's instructions. Mt DNA copy number was quantified using LightCycler 480 SYBR Green I Master (Roche Applied Science) using the following primers on a Roche 480 PCR machine.
MT- ND2:
   F: AAGGGATCCCACTGCACATA (SEQ ID NO:1)
   R: AGTCCTCCTCATGCCCCTAT (SEQ ID NO:2)
RPS18 Nuclear
   F: CCAGAGGTTGCATTTTCCCAAG (SEQ ID NO:3)
   R: TAAGGCCGATAAGGCAAACGAA (SEQ ID NO:4)
*NAD*/*NADH*

NAD+/NADH levels were measured according to the manufacturer's instructions using the NAD/NADH Quantitation Kit (Biovision)

### Spontaneous Oocyte formation (Oocyte formation assay, EFA)

For assessment of spontaneous oocyte formation, each well of a 24- well plate was seeded with 25.000 OSCs, and the number of oocytes formed and released into the medium per well was assessed the second day after seeding as well as the designated time points after NMN treatment.

### Results

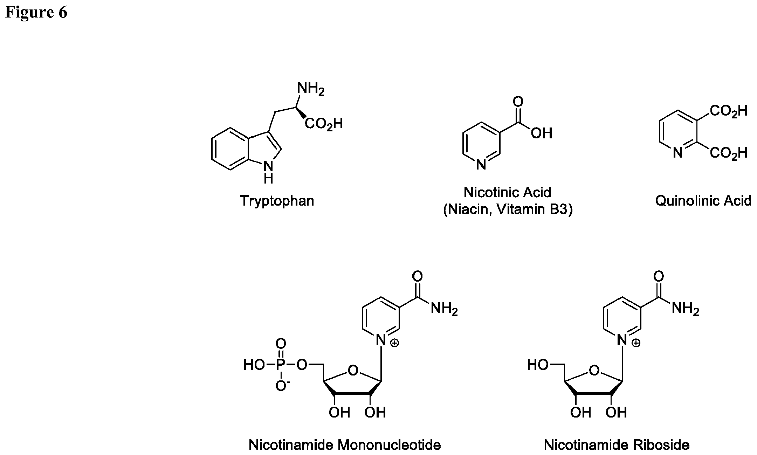

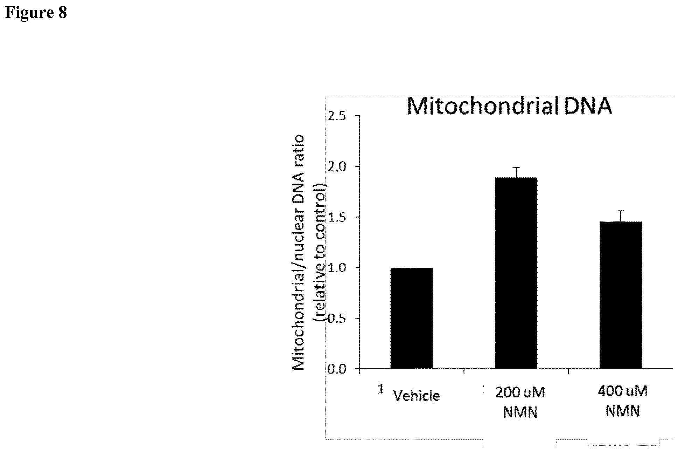

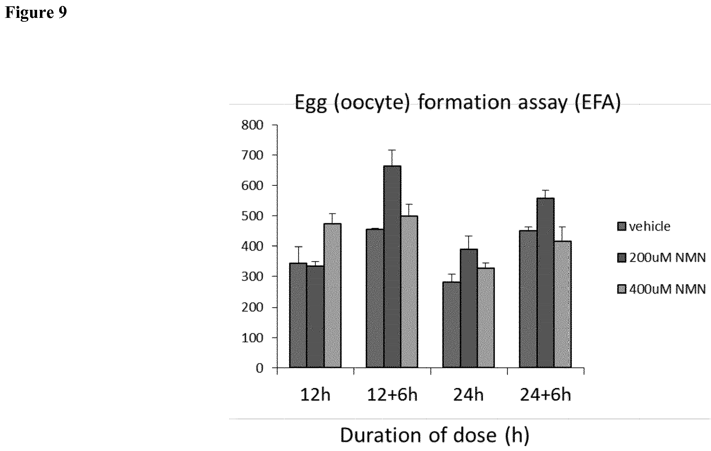

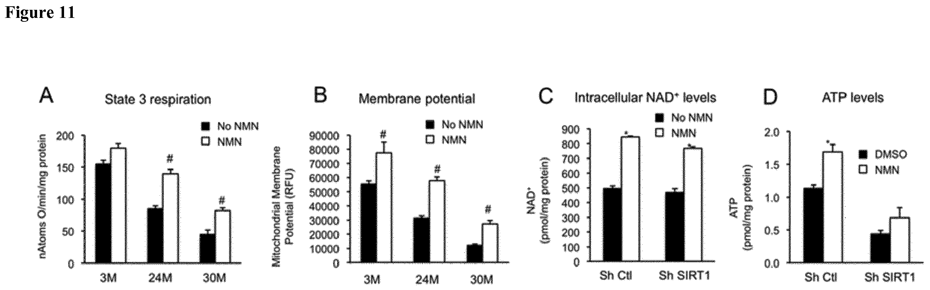

See Figures 5-11.

### Example 3 - Decrease in Weight Gain and Food Consumption in Mice

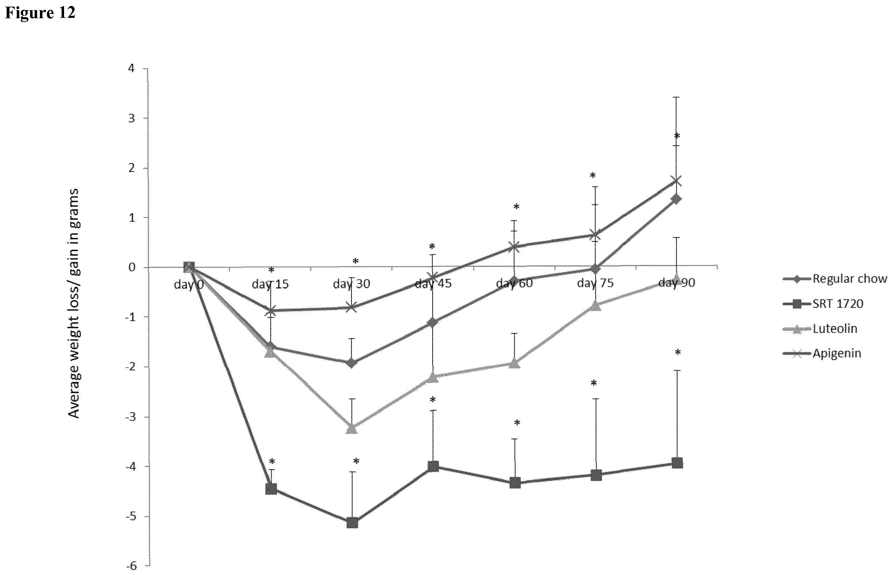

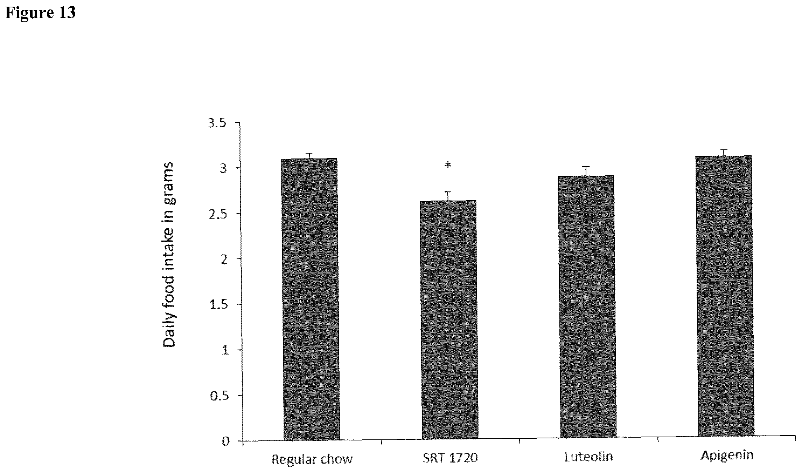

8 months old C57BL/6 mice were obtained from NIA and were maintained on a 12:12 light:dark cycle and provided ad libitum access to water and food. Conditions within rooms were maintained at 21° ± 1 °C with 50% ± 20% relative humidity. Mice were placed on the experimental diets at 8.5 months of age and were maintained for 3 months on the diets. All diets were custom made, ordered from Research Diets: OpenStandard Diet (20 kcal% Protein, 15 kcal% Fat and 65 kcal% Carbohydrate) and the experimental groups consisted of regular OpenStandard Diet, OpenStandard Diet-Apigenin 0.5 gr/kg of food, OpenStandard Diet-Luteolin 0.5 gr/kg of food, OpenStandard Diet-SRT1720 2 gr/kg of food. Food intake was measured weekly and average weight gain every two weeks. Each experimental group consisted of 12 mice. When mice were sacrificed an additional group of young mice (3 months old) were used as controls. See Figure 12 and Figure 13.

### Example 4 - NAD Levels in Varies

### Oogonial stem cell isolation- RNA isolation- Real time PCR

Oogonial stem cells were isolated from dissociated ovaries (3 groups of 4 ovaries each) using a FACS based sorting protocol to purify OSCs free of contaminating oocytes (White et al., Nature Medicine 18(3):413-21, 2012). Cells were lysed in RLT lysis buffer (RNeasy Mini Kit, Qiagen) and RNA extraction was performed according to the manufacturer's instructions. cDNA was generated following the iScript cDNA Synthesis Kit (Bio-Rad) protocol. Transcript levels of Stra8 and Sirt1 were quantified using LightCycler 480 SYBR Green I Master (Roche Applied Science) using the following primers on a Roche 480 PCR machine.
Mm Stra8:
   F: GAGGCCCAGCATATGTCTAAC (SEQ ID NO:5)
   R: GCTCTGGTTCCTGGTTTAATG (SEQ ID NO:6)
MmSirt1:
   F:CTCTGAAAGTGAGACCAGTAGC (SEQ ID NO:7)
   R: TGTAGATGAGGCAAAGGTTCC (SEQ ID NO:8)
Mm Actin:
   F: GATTACTGCTCTGGCTCCTAG (SEQ ID NO:9)
   R: GACTCATCGTACTCCTGCTTG (SEQ ID NO:10)
   *NAD*/*NADH Levels in mouse ovaries*

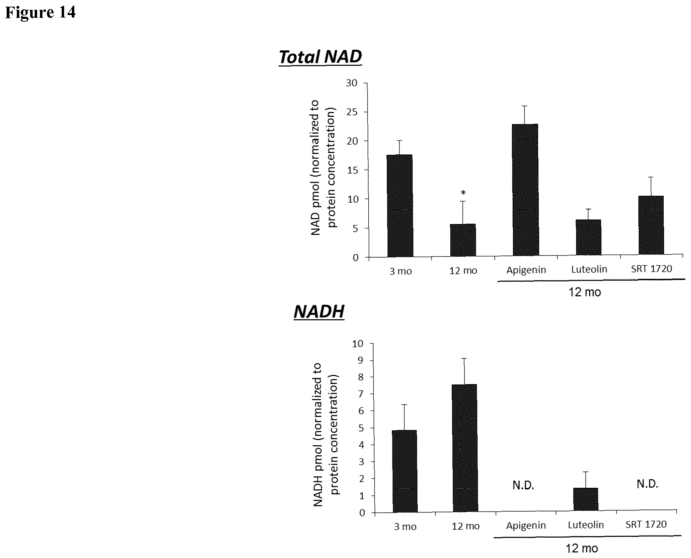

NAD+/NADH levels were measured according to the manufacturer's instructions using the NAD/NADH Quantitation Kit (Biovision). See Figure 14.

### EQUIVALENTS

Those skilled in the art will recognize, or be able to ascertain using no more than routine experimentation, many equivalents of the specific embodiments of the invention described herein. Such equivalents are intended to be encompassed by the following claims.

**In the following aspects and preferred aspects of the invention are further disclosed which are denoted "aspects" and which are numbered for easier reference:**
1. A compound of formula I or a compound of formula **II**: 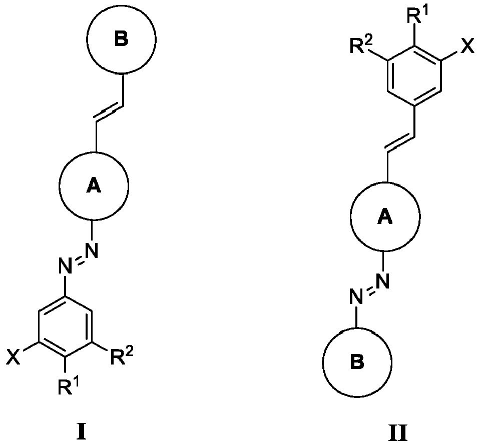 wherein 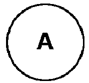
   is an aryl heterocycle diradical; 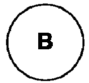
   is heteroaryl;
   X is halo;
   R¹ is hydroxy, alkoxy, or amino; and
   R² is hydroxy, alkoxy, or amino.
2. The compound of aspect 1, wherein 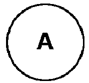 is a diradical of azaindole, benzo(b)thiene, benzimidazole, benzofuran, benzoxazole, benzothiazole, benzothiadiazole, benzotriazole, benzoxadiazole, furan, imidazole, imidazopyridine, indole, indoline, indazole, isoindoline, isoxazole, isothiazole, isoquinoline, oxadiazole, oxazole, purine, pyran, pyrazine, pyrazole, pyridine, pyrimidine, pyrrole, pyrrolo[2,3-d]pyrimidine, pyrazolo[3,4-d]pyrimidine, quinoline, quinazoline, triazole, thiazole, thiobenzene, tetrahydroindole, tetrazole, thiadiazole, thiophene, thiomorpholine, or triazole.
3. The compound of aspect 1, wherein 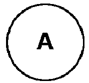 is a diradical of furan, imidazole, isoxazole, isothiazole, oxadiazole, oxazole, pyrrole, triazole, thiazole, tetrazole, thiadiazole, thiophene, or triazole.
4. The compound of aspect 1, wherein 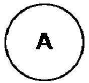 is a diradical of imidazole.
5. The compound of aspect 1, wherein 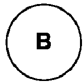 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.
6. The compound of aspect 1, wherein 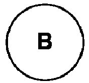 is furanyl, imidazolyl, isoxazolyl, isothiazolyl, oxadiazolyl, oxazolyl, pyrrolyl, triazolyl, thiazolyl, tetrazolyl, thiadiazolyl, thienyl, or triazolyl.
7. The compound of aspect 1, wherein 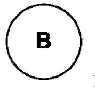 is imidazolyl.
8. The compound of aspect 1, wherein X is bromo.
9. The compound of aspect 1, wherein R¹ is hydroxy.
10. The compound of aspect 1, wherein R² is alkoxy.
11. The compound of aspect 1, wherein R² is methoxy.
12. A compound of formula **III**: 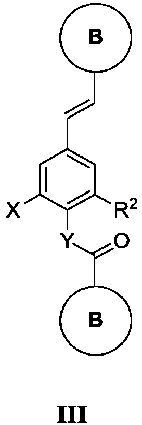 wherein, independently for each occurrence, 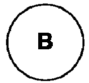
   is heteroaryl;
   X is halo;
   R² is hydroxy, alkoxy, or amino; and
   Y is -O- or -NH-.
13. The compound of aspect 12, wherein 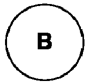 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.
14. The compound of aspect 12, wherein 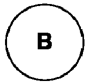 is furanyl, imidazolyl, isoxazolyl, isothiazolyl, oxadiazolyl, oxazolyl, pyrrolyl, triazolyl, thiazolyl, tetrazolyl, thiadiazolyl, thienyl, or triazolyl.
15. The compound of aspect 12, wherein 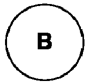 is imidazolyl.
16. The compound of aspect 12, wherein X is bromo.
17. The compound of aspect 12, wherein R² is alkoxy.
18. The compound of aspect 12, wherein R² is methoxy.
19. The compound of aspect 12, wherein Y is -O-.
20. A compound of formula **IV**: 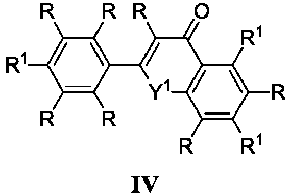 wherein, independently for each occurrence,
   R is -H, halo, aryl, nitro, alkyl, hydroxy, alkoxy, or amino;
   R¹ is hydroxy, alkoxy, or amino; and
   Y¹ is -S-, -O-, or -NH-.
21. The compound of aspect 20, wherein one instance of R is hydroxy.
22. The compound of aspect 20, wherein one instance of R is hydroxy; and the remaining instances of R are -H.
23. The compound of aspect 20, wherein R¹ is hydroxy.
24. The compound of aspect 20, wherein Y¹ is -O-.
25. The compound of aspect 20, provided the compound is not 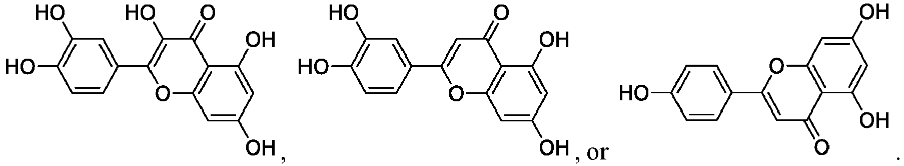
26. A compound of formula **V**: 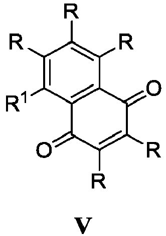 wherein, independently for each occurrence,
   R is -H, halo, aryl, nitro, alkyl, hydroxy, alkoxy, or amino; and
   R¹ is hydroxy, alkoxy, or amino.
27. The compound of aspect 26, wherein R is -H.
28. The compound of aspect 26, wherein R¹ is hydroxy.
29. The compound of aspect 26, provided the compound is not 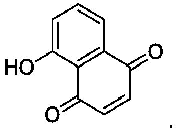
30. A compound of formula **VI**: 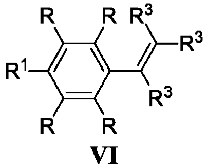 wherein, independently for each occurrence,
   R is -H, halo, aryl, nitro, alkyl, hydroxy, alkoxy, or amino;
   R¹ is hydroxy, alkoxy, or amino;
   R³ is -H, cyano, -CO₂R⁴, or -C(O)N(R⁴)₂; and
   R⁴ is -H or alkyl.
31. The compound of aspect 30, wherein R is -H.
32. The compound of aspect 30, wherein at least one instance of R³ is cyano.
33. The compound of aspect 30, wherein at least two instances of R³ are cyano.
34. The compound of aspect 30, provided the compound is not 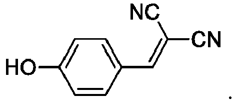
35. A compound of formula **VII**: 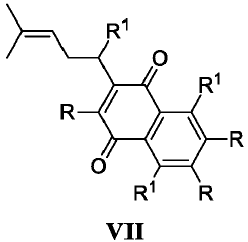 wherein, independently for each occurrence,
   R is -H, halo, aryl, nitro, alkyl, hydroxy, alkoxy, or amino; and
   R¹ is hydroxy, alkoxy, or amino.
36. The compound of aspect 35, wherein R is -H.
37. The compound of aspect 35, wherein R¹ is hydroxy.
38. The compound of aspect 35, provided the compound is not 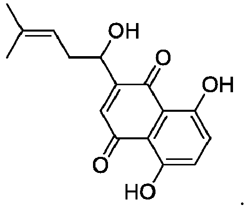
39. A compound of formula **VIII**: 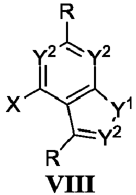 wherein, independently for each occurrence,
   R is -H, halo, aryl, nitro, alkyl, hydroxy, alkoxy, or amino;
   X is halo;
   Y¹ is -O-, -S-, or -NH-; and
   Y² is =N- or =CR-.
40. The compound of aspect 39, wherein R is -H.
41. The compound of aspect 39, wherein X is chloro.
42. The compound of aspect 39, wherein Y¹ is -NH-.
43. The compound of aspect 39, wherein Y² is =N-.
44. The compound of aspect 39, provided the compound is not 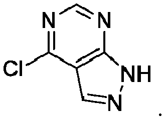
45. A compound of formula **IX**: 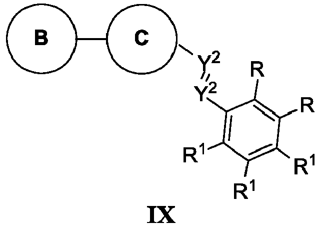 wherein, independently for each occurrence, 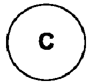
   is a five-membered, unsaturated heterocycle diradical; 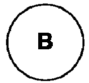
   is heteroaryl;
   R is -H, halo, aryl, nitro, alkyl, hydroxy, alkoxy, or amino;
   R¹ is hydroxy, alkoxy, or amino; and
   Y² is =N- or =CR-.
46. The compound of aspect 45, wherein 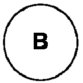 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.
47. The compound of aspect 45, wherein R is -H.
48. The compound of aspect 45, wherein R¹ is hydroxy.
49. The compound of aspect 45, wherein one instance of Y² is =N-.
50. The compound of aspect 45, provided the compound is not 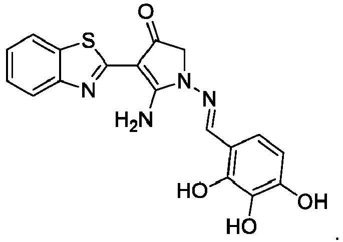
51. A compound of formula **X**: 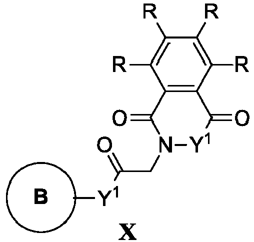 wherein, independently for each occurrence, 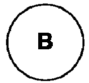
   is heteroaryl;
   R is -H, halo, aryl, nitro, alkyl, hydroxy, alkoxy, or amino;
   Y¹ is -S-, -O-, or -NH-.
52. The compound of aspect 51, wherein 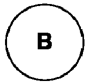 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.
53. The compound of aspect 51, wherein R is -H.
54. The compound of aspect 51, wherein Y¹ is -NH-.
55. The compound of aspect 51, provided the compound is not 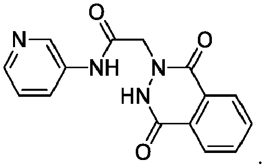
56. A compound of formula **XI**: 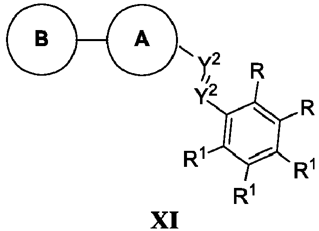 wherein, independently for each occurrence, 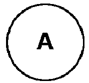
   is an aryl heterocycle diradical; 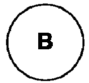
   is heteroaryl;
   R is -H, halo, aryl, nitro, alkyl, hydroxy, alkoxy, or amino;
   R¹ is hydroxy, alkoxy, or amino; and
   Y² is =N- or =CR-.
57. The compound of aspect 56, wherein 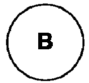 is azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, or triazolyl.
58. The compound of aspect 56, wherein R is -H.
59. The compound of aspect 56, wherein R¹ is hydroxy.
60. The compound of aspect 56, wherein one instance of Y² is =N-.
61. The compound of aspect 56, provided the compound is not 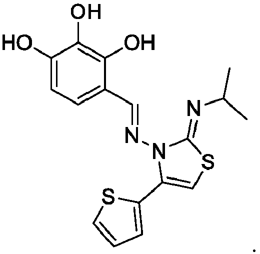
62. A compound of formula **XII:** 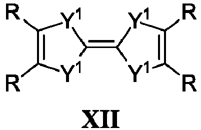 wherein, independently for each occurrence,
   R is -H, halo, aryl, nitro, alkyl, hydroxy, alkoxy, or amino; and
   Y¹ is -S-, -O-, or -NH-.
63. The compound of aspect 62, wherein at least one instance of R is -H.
64. The compound of aspect 62, wherein at least one instance of Y¹ is -S-.
65. The compound of aspect 62, provided the compound is not 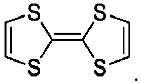
66. A compound of formula **XIII:** 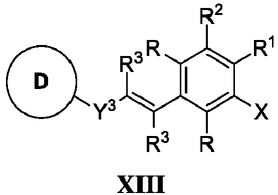 wherein, independently for each occurrence, 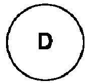
   is aryl or heteroaryl;
   R is -H, halo, aryl, nitro, alkyl, hydroxy, alkoxy, or amino;
   R¹ is hydroxy, alkoxy, or amino;
   R² is hydroxy, alkoxy, or amino;
   R³ is -H, cyano, -CO₂R⁴, or -C(O)N(R⁴)₂;
   X is halo;
   Y³ is a bond, -C(O)-d, -C(O)NH-d, -NH-C(O)-d, or -C(O)NH-CH₂-d; and
   d is a bond to 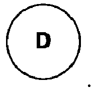
67. The compound of aspect 66, wherein R is -H
68. The compound of aspect 66, wherein R¹ is hydroxy.
69. The compound of aspect 66, wherein R² is alkoxy.
70. The compound of aspect 66, wherein R² is methoxy.
71. The compound of aspect 66, wherein one instance of R³ is cyano.
72. The compound of aspect 66, wherein R³ is -H.
73. The compound of aspect 66, wherein X is bromo.
74. The compound of aspect 66, wherein Y³ is a bond.
75. The compound of aspect 66, wherein Y³ is -C(O)-d.
76. The compound of aspect 66, wherein Y³ is -C(O)NH-d.
77. The compound of aspect 66, wherein Y³ is -NH-C(O)-d.
78. The compound of aspect 66, wherein Y³ is -C(O)NH-CH₂-d.
79. The compound of aspect 66, provided the compound is not 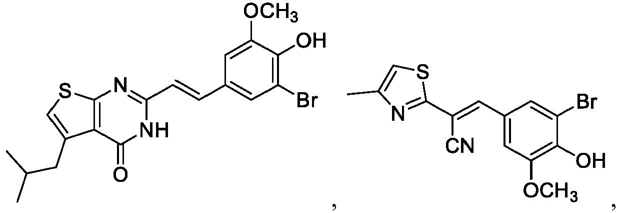 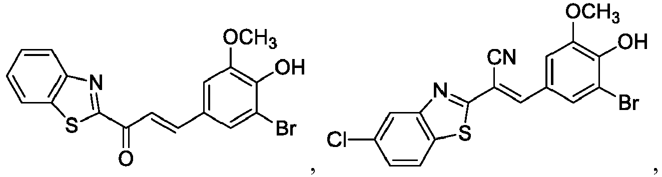 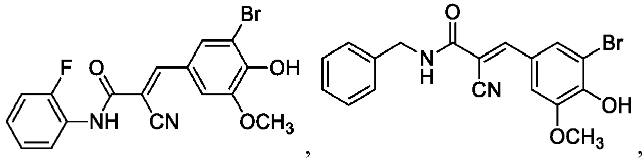 or 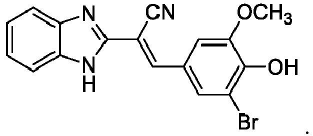
80. A compound of formula **XIV**: 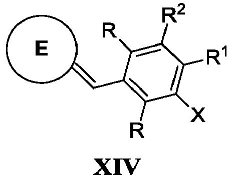 wherein, independently for each occurrence, 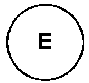
   is a five-membered heterocycle radical;
   R is -H, halo, aryl, nitro, alkyl, hydroxy, alkoxy, or amino;
   R¹ is hydroxy, alkoxy, or amino;
   R² is hydroxy, alkoxy, or amino; and
   X is halo.
81. The compound of aspect 80, wherein R is -H
82. The compound of aspect 80, wherein R¹ is hydroxy.
83. The compound of aspect 80, wherein R² is alkoxy.
84. The compound of aspect 80, wherein R² is methoxy.
85. The compound of aspect 80, wherein X is bromo.
86. The compound of aspect 80, provided the compound is not 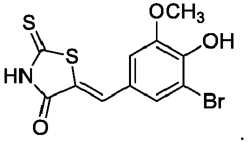
87. A compound of formula **XV**: 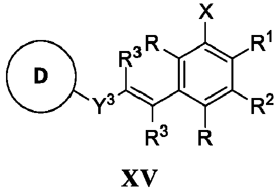 wherein, independently for each occurrence, 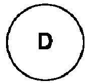
   is aryl or heteroaryl;
   R is -H, halo, aryl, nitro, alkyl, hydroxy, alkoxy, or amino;
   R¹ is hydroxy, alkoxy, or amino;
   R² is hydroxy, alkoxy, or amino;
   R³ is -H, cyano, -CO₂R⁴, or -C(O)N(R⁴)₂;
   X is halo;
   Y³ is a bond, -C(O)-d, -C(O)NH-d, -NH-C(O)-d, or -C(O)NH-CH₂-d; and
   d is a bond to 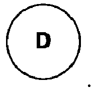
88. The compound of aspect 87, wherein R is -H
89. The compound of aspect 87, wherein R¹ is hydroxy.
90. The compound of aspect 87, wherein R² is alkoxy.
91. The compound of aspect 87, wherein R² is methoxy.
92. The compound of aspect 87, wherein one instance of R³ is cyano.
93. The compound of aspect 87, wherein R³ is -H.
94. The compound of aspect 87, wherein X is bromo.
95. The compound of aspect 87, wherein Y³ is a bond.
96. The compound of aspect 87, wherein Y³ is -C(O)-d.
97. The compound of aspect 87, wherein Y³ is -C(O)NH-d.
98. The compound of aspect 87, wherein Y³ is -NH-C(O)-d.
99. The compound of aspect 87, wherein Y³ is -C(O)NH-CH₂-d.
100. The compound of aspect 87, provided the compound is not 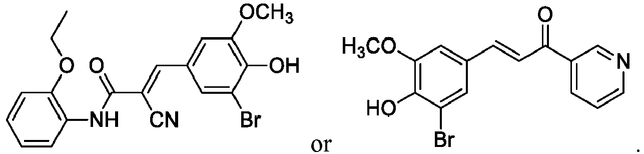
101. A method of inhibiting CD38 in a cell, comprising
   contacting the cell with a therapeutically effective amount of an agent.
102. A method of inhibiting NADase activity in a cell, comprising
   contacting the cell with a therapeutically effective amount of an agent.
103. A method of increasing NAD levels in a cell, comprising
   contacting the cell with a therapeutically effective amount of an agent.
104. A method of increasing NAD⁺ levels in a cell, comprising
   contacting the cell with a therapeutically effective amount of an agent.
105. A method of activating a PARP in a cell, comprising
   contacting the cell with a therapeutically effective amount of an agent.
106. The method of aspect 105, wherein multiple PARPs are activated.
107. A method of treating a subject, comprising
   administering to a subject in need thereof a therapeutically effective amount of an agent.
108. A method of retarding, treating, or preventing an age-related disease in a subject, comprising
   administering to a subject in need thereof a therapeutically effective amount of an agent.
109. A method of retarding aging in a subject, comprising
   administering to a subject in need thereof a therapeutically effective amount of an agent.
110. A method of imparting the benefits of diet to a subject, comprising
   administering to a subject in need thereof a therapeutically effective amount of an agent.
111. A method of imparting the benefits of exercise to a subject, comprising
   administering to a subject in need thereof a therapeutically effective amount of an agent.
112. The method of aspect 108, wherein the age-related disease is selected from the group consisting of neurodegeneration, dementia, a metabolic disease, osteoporosis, an inflammatory disorder, cataracts, bone loss, cardiac dysfunction, cardiovascular diseases, cancer, and a disease resulting from mitochondrial dysfunction.
113. The method of aspect 112, wherein the metabolic disease is selected from the group consisting of type 2 diabetes, shortness of breath, gallbladder disease, hypertension, elevated blood cholesterol levels, cancer, osteoarthritis, other orthopedic problems, reflux esophagitis, snoring, heart trouble, dyslipidemia, coronary heart disease, stroke, hyperinsulinemia, depression, anxiety, gout, fatty liver disease, insulin resistance, pre-diabetes, hypercoagulation, sepsis, inflammatory bowel diseases, dementia, beta-cell dysfunction, sleep apnea, obstructive sleep apnea, hypopnea, and visceral adiposity.
114. The method of any one of aspects 101-113, wherein the agent is a compound of formula **I** or a compound of formula **II**: 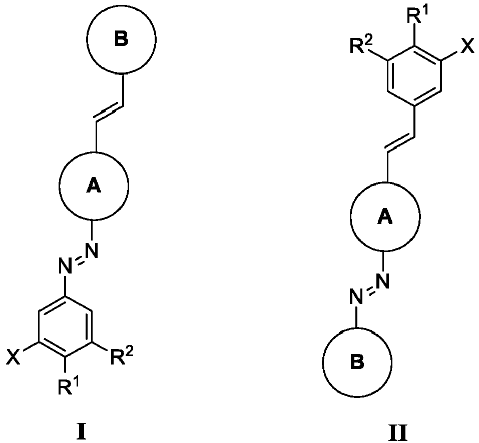 wherein 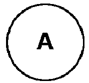
   is an aryl heterocycle diradical; 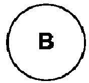
   is heteroaryl;
   X is halo;
   R¹ is hydroxy, alkoxy, or amino; and
   R² is hydroxy, alkoxy, or amino.
115. The method of any one of aspects 101-113, wherein the agent is a compound of formula **III**: 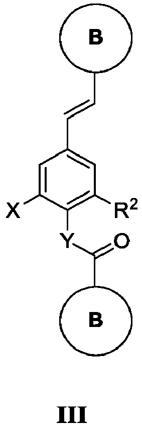 wherein, independently for each occurrence, 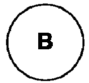
   is heteroaryl;
   X is halo;
   R² is hydroxy, alkoxy, or amino; and
   Y is -O- or -NH-.
116. The method of any one of aspects 101-113, wherein the agent is a compound of formula **IV**: 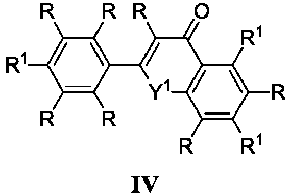 wherein, independently for each occurrence,
   R is -H, halo, aryl, nitro, alkyl, hydroxy, alkoxy, or amino;
   R¹ is hydroxy, alkoxy, or amino; and
   Y¹ is -S-, -O-, or -NH-.
117. The method of any one of aspects 101-113, wherein the agent is a compound of formula **V**: 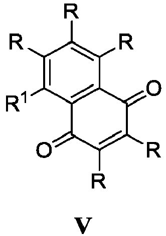 wherein, independently for each occurrence,
   R is -H, halo, aryl, nitro, alkyl, hydroxy, alkoxy, or amino; and
   R¹ is hydroxy, alkoxy, or amino.
118. The method of any one of aspects 101-113, wherein the agent is a compound of formula **VI**: 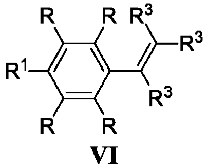 wherein, independently for each occurrence,
   R is -H, halo, aryl, nitro, alkyl, hydroxy, alkoxy, or amino;
   R¹ is R¹ is hydroxy, alkoxy, or amino;
   R³ is -H, cyano, -CO₂R⁴, or -C(O)N(R⁴)₂; and
   R⁴ is -H or alkyl.
119. The method of any one of aspects 101-113, wherein the agent is a compound of formula **VII**: 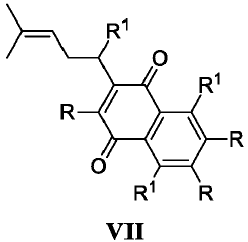 wherein, independently for each occurrence,
   R is -H, halo, aryl, nitro, alkyl, hydroxy, alkoxy, or amino; and
   R¹ is hydroxy, alkoxy, or amino.
120. The method of any one of aspects 101-113, wherein the agent is compound of formula **VIII**: 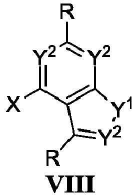 wherein, independently for each occurrence,
   R is -H, halo, aryl, nitro, alkyl, hydroxy, alkoxy, or amino;
   X is halo;
   Y¹ is -O-, -S-, or -NH-; and
   Y² is =N- or =CR-.
121. The method of any one of aspects 101-113, wherein the agent is a compound of formula **IX**: 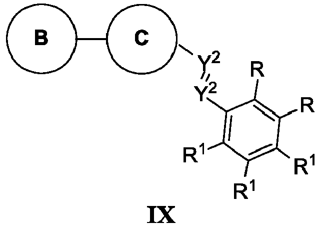 wherein, independently for each occurrence, 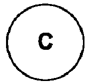
   is a five-membered, unsaturated heterocycle diradical; 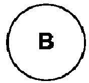
   is heteroaryl;
   R is -H, halo, aryl, nitro, alkyl, hydroxy, alkoxy, or amino;
   R¹ is hydroxy, alkoxy, or amino; and
   Y² is =N- or =CR-.
122. The method of any one of aspects 101-113, wherein the agent is a compound of formula **X**: 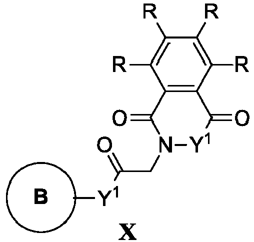 wherein, independently for each occurrence, 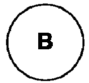
   is heteroaryl;
   R is -H, halo, aryl, nitro, alkyl, hydroxy, alkoxy, or amino;
   Y¹ is -S-, -O-, or -NH-.
123. The method of any one of aspects 101-113, wherein the agent is a compound of formula **XI**: 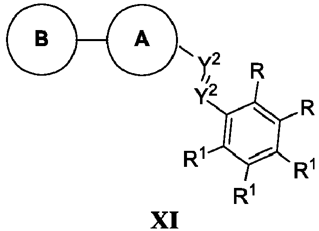 wherein, independently for each occurrence, 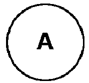
   is an aryl heterocycle diradical; 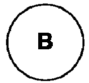
   is heteroaryl;
   R is -H, halo, aryl, nitro, alkyl, hydroxy, alkoxy, or amino;
   R¹ is hydroxy, alkoxy, or amino; and
   Y² is =N- or =CR-.
124. The method of any one of aspects 101-113, wherein the agent is a compound of formula **XII:** 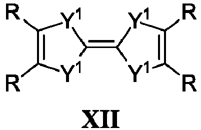 wherein, independently for each occurrence,
   R is -H, halo, aryl, nitro, alkyl, hydroxy, alkoxy, or amino; and
   Y¹ is -S-, -O-, or -NH-.
125. The method of any one of aspects 101-113, wherein the agent is a compound of formula **XIII:** 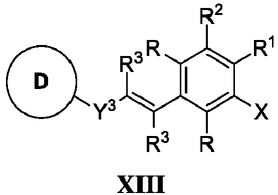 wherein, independently for each occurrence, 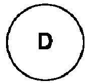
   is aryl or heteroaryl;
   R is -H, halo, aryl, nitro, alkyl, hydroxy, alkoxy, or amino;
   R¹ is hydroxy, alkoxy, or amino;
   R² is hydroxy, alkoxy, or amino;
   R³ is -H, cyano, -CO₂R⁴, or -C(O)N(R⁴)₂;
   X is halo;
   Y³ is a bond, -C(O)-d, -C(O)NH-d, -NH-C(O)-d, or -C(O)NH-CH₂-d; and
   d is a bond to 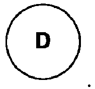
126. The method of any one of aspects 101-113, wherein the agent is a compound of formula **XIV**: 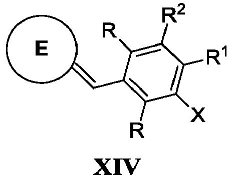 wherein, independently for each occurrence, 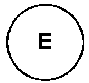
   is a five-membered heterocycle radical;
   R is -H, halo, aryl, nitro, alkyl, hydroxy, alkoxy, or amino;
   R¹ is hydroxy, alkoxy, or amino;
   R² is hydroxy, alkoxy, or amino; and
   X is halo.
127. The method of any one of aspects 101-113, wherein the agent is a compound of formula **XV**: 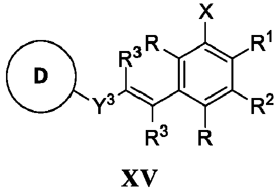 wherein, independently for each occurrence, 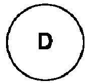
   is aryl or heteroaryl;
   R is -H, halo, aryl, nitro, alkyl, hydroxy, alkoxy, or amino;
   R¹ is hydroxy, alkoxy, or amino;
   R² is hydroxy, alkoxy, or amino;
   R³ is -H, cyano, -CO₂R⁴, or -C(O)N(R⁴)₂;
   X is halo;
   Y³ is a bond, -C(O)-d, -C(O)NH-d, -NH-C(O)-d, or -C(O)NH-CH₂-d; and
   d is a bond to 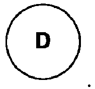
128. The method of any one of aspects 101-113, wherein the agent is 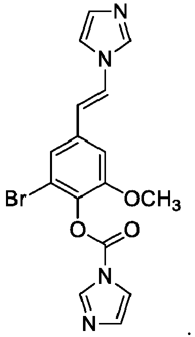
129. The method of any one of aspects 101-113, wherein the agent is selected from the group consisting of 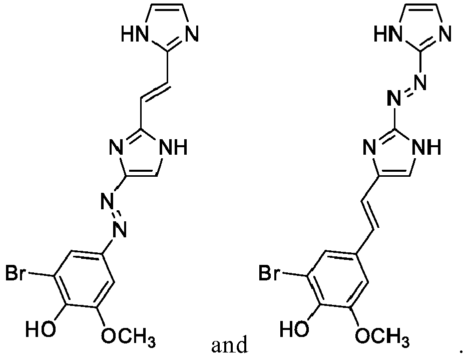
130. The method of any one of aspects 101-113, wherein the agent is selected from the group consisting of 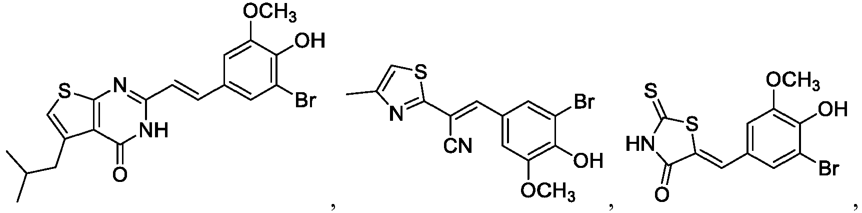 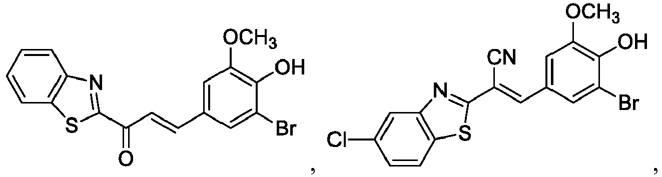 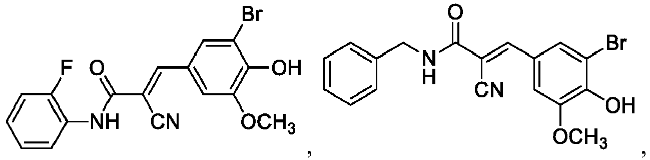 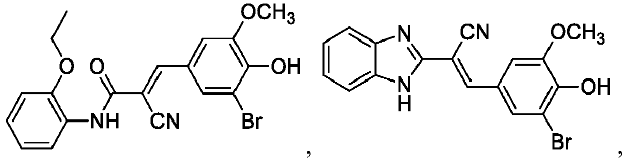 and 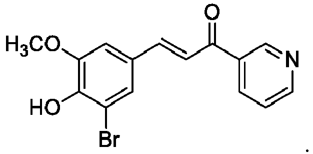
131. The method of any one of aspects 101-113, wherein the agent is selected from the group consisting of 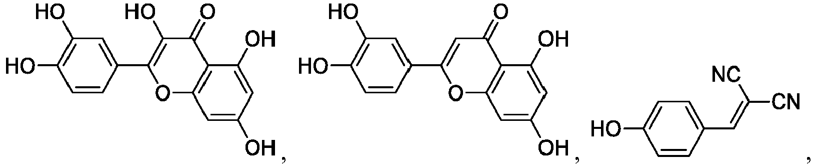 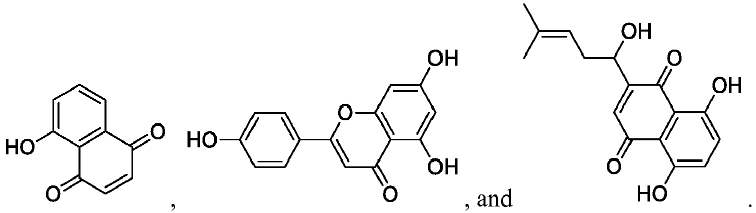
132. The method of any one of aspects 101-113, wherein the agent is selected from the group consisting of 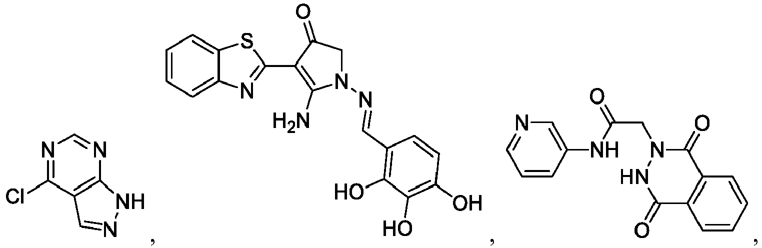 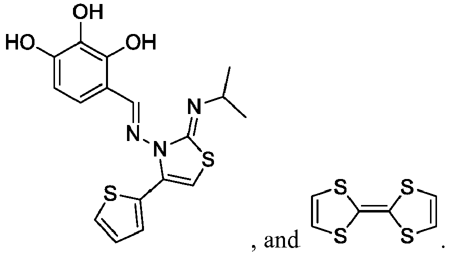
133. The method of any one of aspects 101-113, wherein the agent is selected from the group consisting of α-lineolic acid, lineolic acid, stearic acid, elaidic acid, arachidonic acid, oleic acid, and palmitoleic acid.
134. The method of any one of aspects 101-113, wherein the agent is selected from the group consisting of tryptophan, quinolinic acid, nicotinamide, nicotinamide mononucleotide, nicotinamide riboside, and nicotinic acid.

## Claims

1. An agent for use in:
i) retarding, treating, or preventing an age-related disease in a subject; or
ii) retarding aging in a subject; or
iii) imparting the benefits of diet or exercise to a subject,
wherein the agent is selected from nicotinamide riboside, tryptophan, quinolinic acid, nicotinamide, nicotinamide mononucleotide, and nicotinic acid.

2. The agent for use of claim 1, wherein the use is retarding, treating, or preventing an age-related disease in a subject.

3. The agent for use of claim 2, wherein the age-related disease is selected from neurodegeneration, dementia, a metabolic disease, osteoporosis, an inflammatory disorder, cataracts, bone loss, cardiac dysfunction, cardiovascular diseases, cancer, and a disease resulting from mitochondrial dysfunction.

4. The agent for use of claim 3, wherein the age-related disease is a metabolic disease; and the metabolic disease is selected from type 2 diabetes, shortness of breath, gallbladder disease, hypertension, elevated blood cholesterol levels, cancer, osteoarthritis, other orthopedic problems, reflux esophagitis, snoring, heart trouble, dyslipidemia, coronary heart disease, stroke, hyperinsulinemia, depression, anxiety, gout, fatty liver disease, insulin resistance, pre-diabetes, hypercoagulation, sepsis, inflammatory bowel diseases, dementia, beta-cell dysfunction, sleep apnea, obstructive sleep apnea, hypopnea, and visceral adiposity.

5. The agent for use of claim 3, wherein the age-related disease is an inflammatory disorder; and the inflammatory disorder is selected from arthritis, Crohn's disease, rheumatoid arthritis, asthma, atherosclerosis, coronary heart disease, reperfusion injury from a heart attack, reperfusion injury from a stroke, ulcerative colitis, and active inflammatory bowel disease (IBD).

6. The agent for use of claim 1, wherein the use is retarding aging in a subject.

7. The agent for use of claim 1, wherein the use is imparting the benefits of diet or exercise to a subject.

8. The agent for use of claim 7, wherein the benefits are selected from prevention of obesity, prevention of liver steatosis, prevention of cardiovascular disease, prevention of insulin resistance, and prevention of type II diabetes.

9. The agent for use of any one of claims 1-8, wherein the agent is nicotinamide riboside.

10. The agent for use of any one of claims 1-9, wherein the agent is administered as a food supplement.

11. The agent for use of any one of claims 1-9, wherein the agent is a component of a multi-vitamin complex.

12. The agent for use of any one of claims 1-11, wherein the agent is administered orally.


SEQUENCE LISTING

<110> PRESIDENT AND FELLOWS OF HARVARD COLLEGE
      MAYO FOUNDATION FOR MEDICAL EDUCATION AND RESEARCH

<120> SMALL MOLECULE CD38 INHIBITORS AND METHODS OF USING SAME

<130> HA20222PCTEPD1

<140> Divisional to EP 12 804 280.1
<141> 2012-04-13

<150> PCT/US2012/033571
<151> 2012-04-13

<150> 61/502,588
<151> 2011-06-29

<160> 10

<170> PatentIn version 3.5

<210> 1
<211> 20
<212> DNA
<213> Artificial Sequence

<220>
<221> source
<223> /note="Description of Artificial Sequence: Synthetic
      primer"

<400> 1
aagggatccc actgcacata                                                20


<210> 2
<211> 20
<212> DNA
<213> Artificial Sequence

<220>
<221> source
<223> /note="Description of Artificial Sequence: Synthetic
      primer"

<400> 2
agtcctcctc atgcccctat                                                20


<210> 3
<211> 22
<212> DNA
<213> Artificial Sequence

<220>
<221> source
<223> /note="Description of Artificial Sequence: Synthetic
      primer"

<400> 3
ccagaggttg cattttccca ag                                             22

<210> 4
<211> 22
<212> DNA
<213> Artificial Sequence

<220>
<221> source
<223> /note="Description of Artificial Sequence: Synthetic
      primer"

<400> 4
taaggccgat aaggcaaacg aa                                                22


<210> 5
<211> 21
<212> DNA
<213> Artificial Sequence

<220>
<221> source
<223> /note="Description of Artificial Sequence: Synthetic
      primer"

<400> 5
gaggcccagc atatgtctaa c                                                 21


<210> 6
<211> 21
<212> DNA
<213> Artificial Sequence

<220>
<221> source
<223> /note="Description of Artificial Sequence: Synthetic
      primer"

<400> 6
gctctggttc ctggtttaat g                                                 21


<210> 7
<211> 22
<212> DNA
<213> Artificial Sequence

<220>
<221> source
<223> /note="Description of Artificial Sequence: Synthetic
      primer"

<400> 7
ctctgaaagt gagaccagta gc                                                22


<210> 8
<211> 21
<212> DNA
<213> Artificial Sequence

<220>

<221> source
<223> /note="Description of Artificial Sequence: Synthetic
      primer"

<400> 8
tgtagatgag gcaaaggttc c                                                 21


<210> 9
<211> 21
<212> DNA
<213> Artificial Sequence

<220>
<221> source
<223> /note="Description of Artificial Sequence: Synthetic
      primer"

<400> 9
gattactgct ctggctccta g                                                 21


<210> 10
<211> 21
<212> DNA
<213> Artificial Sequence

<220>
<221> source
<223> /note="Description of Artificial Sequence: Synthetic
      primer"

<400> 10
gactcatcgt actcctgctt g                                                 21